# EUROPEAN PATENT SPECIFICATION

(11) **EP 3 215 630 B1**
(45) Date of publication and mention of the grant of the patent: **19.02.2020**
(21) Application number: 15794123.8
(22) Date of filing: 06.11.2015
(51) Int. Cl.: C12Q 1/00, C12Q 1/04, C12Q 1/06, C12N 9/14

(54) **ANALYTICAL AND DIAGNOSTIC METHODS UTILIZING SHIGELLA FLEXNERI APYRASE**
ANALYTISCHE UND DIAGNOSTISCHE VERFAHREN MIT VERWENDUNG VON SHIGELLA-FLEXNERI-APYRASE
PROCÉDÉS ANALYTIQUES ET DIAGNOSTIQUES UTILISANT L'APYRASE DE SHIGELLA FLEXNERI

(30) Priority: 07.11.2014 SE 1451332
(43) Date of publication of application: 13.09.2017
(73) Proprietor: ApiRays Bioscience AB, 114 31 Stockholm (SE)
(72) Inventor: ASALAPURAM, Pavankumar, S-14156 Huddinge (SE); RUSSOM, Aman, S-17076 Solna (SE)
(74) Representative: Novitas Patent AB
(86) International application number: PCT/EP2015/075924
(87) International publication number: WO 2016/071497

(56) References cited:
- EP-A2- 0 781 851
- WO-A1-94/12211
- WO-A1-2007/061293
- WO-A1-2008/015427
- WO-A1-2011/102808
- GB-A- 2 261 878
- BABU M M ET AL: "Shigella apyrase - a novel variant of bacterial acid phosphatases?", FEBS LETTERS, ELSEVIER, AMSTERDAM, NL, vol. 512, no. 1-3, 13 February 2002 (2002-02-13), pages 8-12, XP004341340, ISSN: 0014-5793, DOI: 10.1016/S0014-5793(02)02287-1

## Description

### TECHNICAL FIELD

The present invention relates to analytical and diagnostic methods where contaminating nucleotides are an issue. In particular, the present invention relates to determining or quantifying the amount of ATP present in a sample that may contain contaminating nucleotides, as well as reagents for use in such methods and production of such reagents.

### BACKGROUND

Adenosine triphosphate (ATP) is a molecule present in all living cells. Since the concentration of ATP is fairly constant in the cell, measurement of ATP content in a sample can be used as a proxy to determine the number of viable cells. Sensitive bioluminescent assays for measuring ATP based on luciferase/luciferin are known, see e.g. US3745090. Luciferase (e.g. from firefly) is a euglobulin protein that catalyses the oxidative decarboxylation of luciferin using ATP and molecular oxygen to yield oxyluciferin, a highly unstable, single-stage excited compound that emits light upon relaxation to its ground state. This reaction emits light proportional to ATP concentration in the reaction mixture, and by measuring the intensity of the emitted light it is possible to continuously monitor the concentration of ATP present.

In many cases, samples to be analyzed for ATP content contain contaminating ATP either extracellularly or present in a contaminating type of cells. For instance, if the number of bacteria is to be quantitated in any clinical or biological sample, any ATP contained in host cells present in the sample will interfere with the measurement. In many applications, the contaminating cells may be selectively lysed and the ATP released, resulting in that all the contaminating ATP is in extracellular form, see e. g. US4303752 or US20110076706. In other instances, ATP analogues different from ATP may be present in a sample and interfere with ATP measurement.

The contaminating extracellular ATP, otherwise unwanted ATP or ATP analogues can be reduced or eliminated by hydrolyzing them with an enzyme called apyrase (ATP-diphosphohydrolase, E-type ATPase, ATPDase, NTDase EC 3.6.1.5). Apyrase is frequently used in methods for determining bacterial ATP in the presence of mammalian cells, where the mammalian cells are first selectively lysed and apyrase used to degrade extracellular ATP leaving the bacterial ATP unaffected. After completion of the reaction, the apyrase can be inactivated and the intracellular ATP of bacterial cells is released to measure bacterial ATP by the addition of luciferin/luciferase. Light emission is measured before and after the addition of a known amount of ATP standard, as internal control. The bacterial ATP (in moles) is calculated by multiplying the ratio of the light before and after adding the ATP standard with the amount of added standard. Typically bacterial cells contain around 1 attomole of ATP per cell, making it possible to estimate the number of bacterial cells from the amount of ATP detected. It is an object of the present invention to provide improvements for such analytical and diagnostic methods.

In this context, the most commonly used apyrase is *Solanum tuberosum* apyrase (STA; commonly known as potato apyrase, SEQ ID NO: 9). STA exists in several isoforms and each isoform differs in ATP-degradation activity. However, the efficiency of STA in the above methods is limited by the accumulation of ADP and uncharacterized ATP-analogues in the degradation reaction when using STA. Accumulation of such contaminants inhibit the ATP degradation capability allowing some of the contaminating ATP to remain intact, which in turn limits the sensitivity of the ATP determination assays and also increases the background signal. For a discussion on the limitations of STA, see WO199402816.

Further, most embodiments of the DNA sequencing method pyrosequencing (see e.g. international patent applications PCT/GB1997/002631 and PCT/GB1997/003518, or US patent applications US2013/0045876 and US2013/0189717) also rely on quantitation of ATP, usually by the luciferase/luciferin assay. At the end of every sequencing cycle, all the unincorporated nucleotides and excess ATP must be eliminated by e.g. apyrase. As detailed above, the apyrases presently used in DNA sequencing applications have problems in achieving complete degradation due to the quality of enzyme (contamination of NDP kinase), substrate specificity and batch-to-batch variations that not only results in *drop off* and nonlinear peaks but also affects DNA sequencing of long strands. It is an object of the present invention to provide better elimination of ATP, its analogues as well as other di- or triphosphate nucleotides to e.g. improve DNA sequencing.

WO 94/12211 and GB 2 261878 disclose recombinant *Shigella flexneri*-apyrases, related sequences, vector and host cells. Babu et al. (FEBS LETTERS, vol 512, no. 1-3 (2002), pages 8-12) discloses the amino-acid sequence of the *Shigella flexneri*-apyrase.

EP 0781851, WO 2008/015427, WO 2007/061293 and WO 2011/102808 disclose methods of ATP determination, pyrosequencing, sequencing by synthesis and the like, where enzymatic degradation of unwanted nucleotides is performed.

### DEFINITIONS

The following terms and words have the meanings as defined below in the context of the present disclosure.

The words *comprise* or *comprising* is to be interpreted in a non-limiting fashion, such as to include or to contain; have as a component.

*Shigella flexneri* apyrase (SFA) is defined as apyrase derived from *Shigella flexneri,* at any suitable degree of purity. The SFA may be produced by non-recombinant means or by recombinant DNA technology. Recombinant *Shigella flexneri* apyrase is abbreviated rSFA herein. The native SFA has the sequence according to SEQ ID NO: 10. The SFA according to the definition has apyrase activity substantially similar to the rSFA according to SEQ ID NO. 4 or SFA according to SEQ ID NO: 10. Apyrase activity refers to capacity to catalyse hydrolysis of nucleoside triphosphates to nucleoside diphosphates, and hydrolysis of nucleoside diphosphates to nucleoside monophosphates.

*Sequence identity* expressed in percentage is defined as the value determined by comparing two optimally aligned sequences over a comparison window, wherein a portion of the sequence in the comparison window may comprise additions or deletions (i.e., gaps) as compared to the reference sequence (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Unless indicated otherwise, the comparison window is the entire length of the sequence being referred to. In this context, optimal alignment is the alignment produced by the BLASTP algorithm as implemented online by the US National Center for Biotechnology Information (see The NCBI Handbook [Internet], Chapter 16), with the following input parameters: Word length=3, Matrix=BLOSUM62, Gap cost=11, Gap extension cost=1.

*ATP analogues* refer to compounds with structural and functional similarity to ATP which compete with ATP for binding to enzymes that specifically interact with ATP. In the present context, the term only applies to compounds that are substrates for SFA as defined above. Preferably, the term refers to compounds that can substitute ATP as substrate for luciferases, such as firefly luciferase. In preferable embodiments, the term includes nucleoside diphosphates and nucleoside triphosphates. In more preferable embodiments, the term refers to adenosine diphosphate, deoxyadenosine alpha-thio triphosphate, adenosine tetra-phosphate, deoxyadenosine triphosphate, deoxyadenosine diphosphate, guanosine triphosphate, guanosine diphosphate, deoxyguanosine triphosphate, deoxyguanosine diphosphate, thymidine triphosphate, deoxythymidine triphosphate, thymidine diphosphate, deoxythymidine triphosphate, cytidine diphosphate, deoxycytidine triphosphate, cytidine triphosphate, deoxycytidine diphosphate, uridine diphosphate and uridine triphosphate. In most preferable embodiments, ATP analogues refers to: adenosine diphosphate, deoxyadenosine alpha-thio triphosphate, adenosine tetra-phosphate, deoxyadenosine triphosphate, deoxyadenosine diphosphate, guanosine triphosphate, guanosine diphosphate, deoxyguanosine triphosphate and deoxyguanosine diphosphate.

### SUMMARY OF THE INVENTION

Briefly, the present invention relates to the following items. The subject matter disclosed in the items below should be regarded disclosed in the same manner as if the subject matter were disclosed in patent claims. The scope of patent protection is determined by the scope of the appended claims. The description also discloses :
1. A method, comprising the steps of
   a. providing a sample containing contaminating nucleoside diphosphates and/or nucleoside triphosphates, such as ATP and/or ATP analogues including deoxyribonucleoside triphosphates;
   b. reducing the amount of the contaminating nucleoside diphosphates and/or nucleoside triphosphates in the sample with an apyrase enzyme, wherein said apyrase enzyme is a *Shigella flexneri* apyrase; and
   c. performing an analysis of the sample, wherein said analysis comprises an assay that would have been affected by the contaminating nucleoside diphosphates and/or nucleoside triphosphates had they not been reduced in step b.
2. The method according to item 1, being a method for determining the amount of ATP in a sample, comprising the steps of:
   a. reducing the amount of contaminating ATP and/or ATP analogues in the sample by degradation with an apyrase enzyme;
   b. making the ATP to be determined available for determination; and
   c. determining the amount of ATP to be determined in the sample, wherein the apyrase enzyme is a *Shigella flexneri* apyrase.
3. The method according to item 2, wherein the method is for determining the amount of ATP present in a first population of cells in a sample, comprising the steps of:
   a. reducing the amount of contaminating ATP and/or ATP analogues in the sample by degradation with an apyrase enzyme;
   b. liberating the ATP to be determined from the first population of cells; and
   c. determining the amount of liberated ATP; wherein the apyrase enzyme in step (a) is a *Shigella flexneri* apyrase.
4. The method according to item 3, wherein the liberation step (b) involves lysis of the first population of cells.
5. The method according to any of items 3-4, wherein the first population of cells comprises bacterial cells.
6. The method according to any of items 1-5, wherein the sample is a biological sample from an animal.
7. The method according to item 6, wherein the sample is a biological sample from a human.
8. The method according to any of items 1-7, wherein the sample is a blood sample, a plasma sample, a serum sample, a urine sample, a faecal sample, or a swab from a patient.
9. The method according to any of items 2-8, wherein at least a fraction of the contaminating ATP is present in a second population of cells, and the reduction step (a) is preceded by a step of selective liberation of ATP from the second population of cells.
10. The method according to item 9, wherein the second population of cells are host cells from an animal from which the sample is derived.
11. The method according to any of items 1-10, where the method comprises the step of adding an apyrase inhibitor after the reduction step.
12. The method according to item 11, wherein the apyrase inhibitor comprises ortho-vanadate or Mg²⁺, preferably ortho-vanadate.
13. The method according to item 12, wherein ortho-vanadate is present in the sample at a concentration of at least 0.1 mM, preferably 0.2-25 mM, more preferably 0.5-20 mM, or Mg²⁺ is present in the sample at a concentration of at least 25 mM, preferably 30-200 mM, more preferably 30-150 mM.
14. The method according to any of items 1-5 or 11-13, wherein the sample is a cell culture medium sample, food sample, a beverage sample, a pharmaceutical sample, a sewage sample, an environmental sample such as a swab from a surface, or a drinking water sample.
15. The method according to any of items 3-14, comprising step (d) of calculating the number or concentration of viable cells of the first population present in the sample from the amount of ATP determined in step (c).
16. The method according to item 1, wherein the method is a sequencing-by-synthesis procedure, the contaminating nucleotides comprise excess dNTPs or analogues thereof present after a completed sequencing cycle, and the analysis performed on the sample is a sequence readout.
17. The method according to item 1 or 16, wherein the method is for doing pyrosequencing and comprises the steps of:
   a. performing a pyrosequencing reaction comprising addition of a nucleoside triphosphate;
   b. converting the pyrophosphate released in step (a) into ATP via an enzymatic reaction;
   c. determining the amount of ATP formed in step (b);
   d. degrading unincorporated nucleoside triphosphate from step (a) and ATP formed in step (b) with an apyrase enzyme;
   e. repeating the steps a-d at least once;
   wherein the apyrase enzyme is a *Shigella flexneri* apyrase.
18. The method according to any of items 2-15, wherein the determination of the amount of ATP is performed with a bioluminescent assay.
19. The method according to item 18, wherein the determination of the amount of ATP is performed with bioluminescent assay utilizing luciferin and luciferase.
20. The method according to item 19, wherein luciferase is provided in a composition comprising a luciferase and an apyrase inhibitor comprising ortho-vanadate, wherein the concentration of ortho-vanadate is preferably at least 0.2 mM, more preferably at least 1 mM, yet more preferably 1-3000 mM, most preferably 2-200 mM.
21. The method according to any of the preceding items, wherein the *Shigella flexneri* apyrase comprises an amino-acid sequence with at least 80%, more preferably at least 85%, even more preferably at least 90%, yet more preferably at least 95%, still more preferably at least 97%, most preferably 100% sequence identity to SEQ ID NO: 10.
22. The method according to any of the preceding items, wherein the *Shigella flexneri* apyrase comprises an amino-acid sequence according to SEQ ID NO: 4.
23. The method according to any of the preceding items, wherein *Shigella flexneri* apyrase is provided in a buffer having a pH in the range of 6-9, preferably 7-8, and an ionic strength of at least 300 mM, wherein the ionic strength is preferably 300-1000 mM, more preferably 400-800 mM, even more preferably 500-800 mM..
24. A use of a *Shigella flexneri* apyrase for degrading contaminating nucleoside triphosphates or nucleoside disphosphates in an analytical method.
25. The use according to item 24, wherein the contaminating nucleosides comprise contaminating ATP and/or ATP analogues, and the analytical method comprises an assay for measuring ATP.
26. The use according to item 25, wherein the assay for measuring ATP is a bioluminescent assay.
27. A use of a *Shigella flexneri* apyrase in a sequencing by synthesis assay, preferably a pyrosequencing assay.
28. The use according to any of items 24-27, wherein the apyrase comprises an amino-acid sequence with at least 80%, more preferably at least 85%, even more preferably at least 90%, yet more preferably at least 95%, still more preferably at least 97%, most preferably 100% sequence identity to SEQ ID NO: 10.
29. The use according to any of items 24-28, wherein the apyrase comprises an amino-acid sequence with at least 95 %, preferably at least 96%, more preferably at least 97%, yet more preferably at least 98%, still more preferably at least 99%, most preferably complete sequence identity to SEQ ID NO: 4 and having apyrase activity.

### BRIEF DESCRIPTION OF THE DRAWINGS

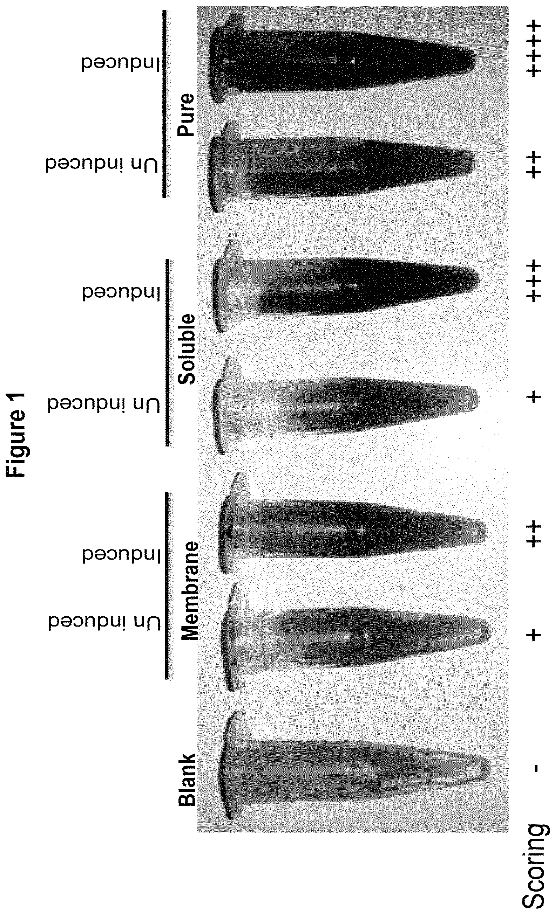

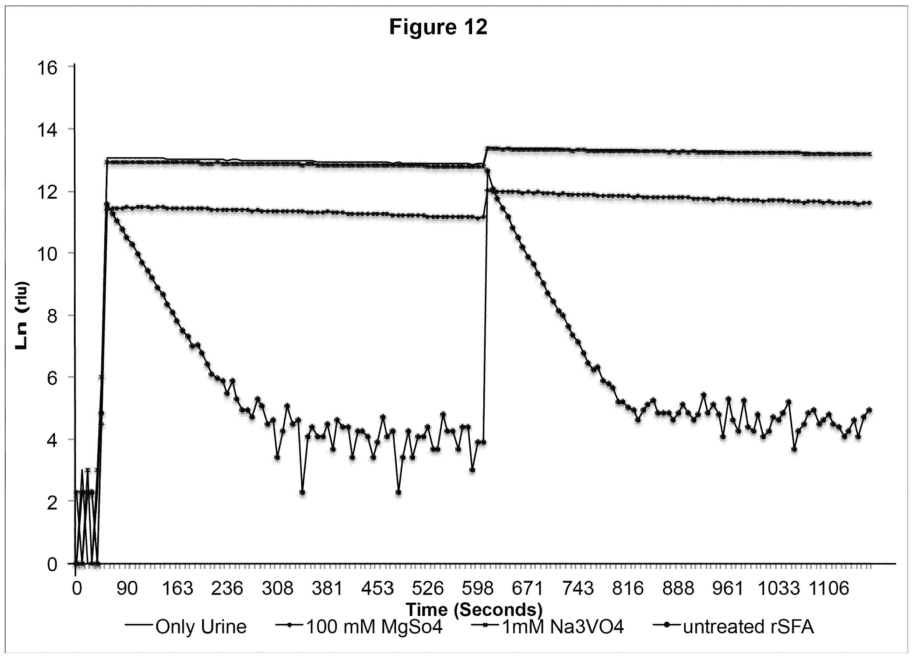

**Figure 1****. Colorimetric evaluation of recombinant *Shigella flexineri* apyrase (rSFA).** The overexpressed rSFA was tested colorimetrically using ammonium molybdate and ferrous ammonium sulphate to check the localization, quality and efficiency. Sub-cellular fractions of *E. coli* BL21-A1 containing apyrase enzyme were tested and the optical densities (O.D.) of these fractions were measured in colorimeter to make a qualitative and/or quantitative correlation. Here, the inventors have made a qualitative comparison to localize the expression of apyrase enzyme among certain sub-cellular fractions of the developed clone and show its high activity through colorimetric analysis.

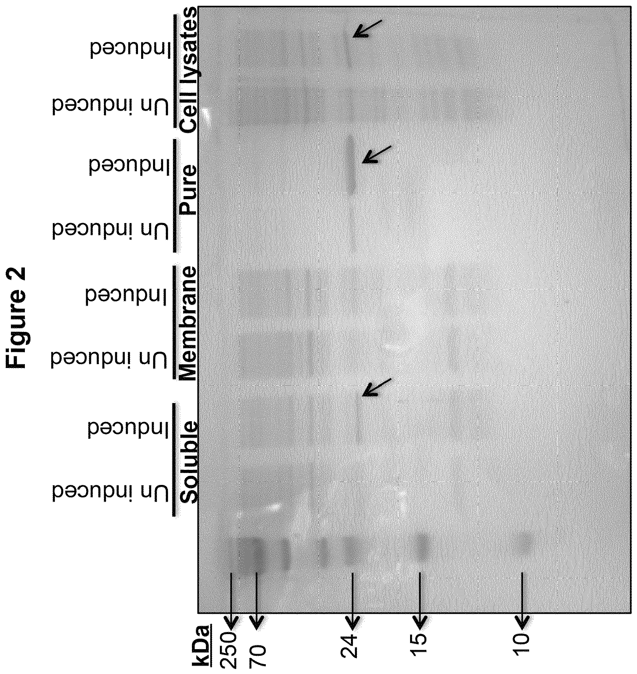

**Figure 2****. Sub-cellular localization of rSFA enzyme expression.** Sub-cellular fractions of the *E*. *coli* BL21-A1 expression vector bearing the *Shigella flexineri* apyrase (SFA) gene with 6-histidines in pBL plasmid were resolved on 10% SDS-PAGE to check the expression of the enzyme. Expression of rSFA was found 25 kDa in fractions of induced soluble samples, which was used to purify the apyrase enzyme. The non-induced and induced samples of purified fractions and the whole cell lysates, respectively. This experiment showed that the enzyme is localized in the soluble fractions of the expression host.

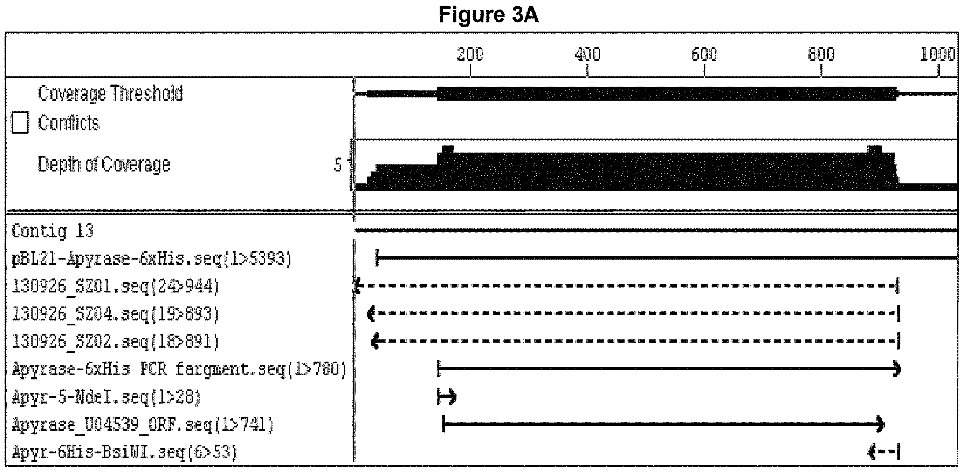

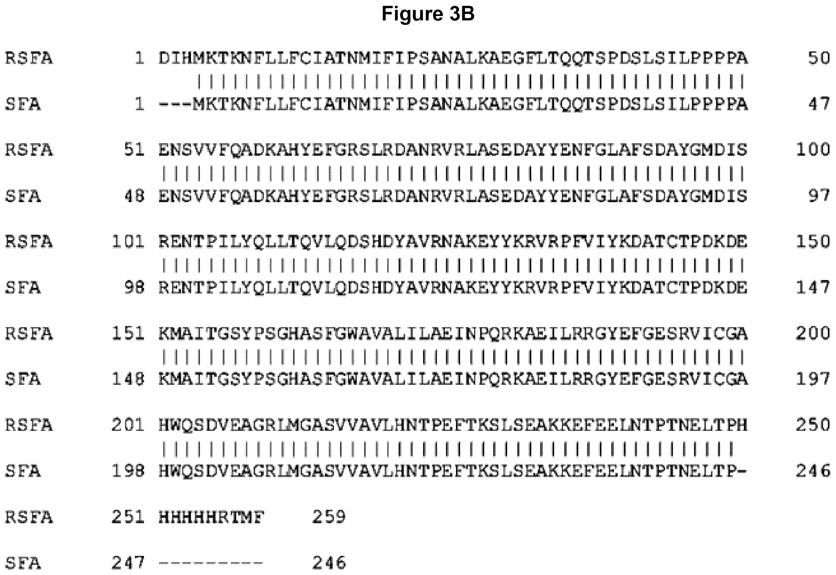

**Figure 3****. Sequence analysis of rSFA**
   The transformed clone of rSFA bearing apyrase gene with 6x Histidines was sequenced by Sanger's method. The cloned fragment contained 780 homologous bases (Fig. 3A) of 1134 nucleotides of virulent SFA. Fig 3B illustrates the similarity between the native SFA reference sequence (SEQ ID NO: 10, termed "SFA") and rSFA (SEQ ID NO: 4, termed "rSFA". The sequences differ only at the N- and the C-termini.

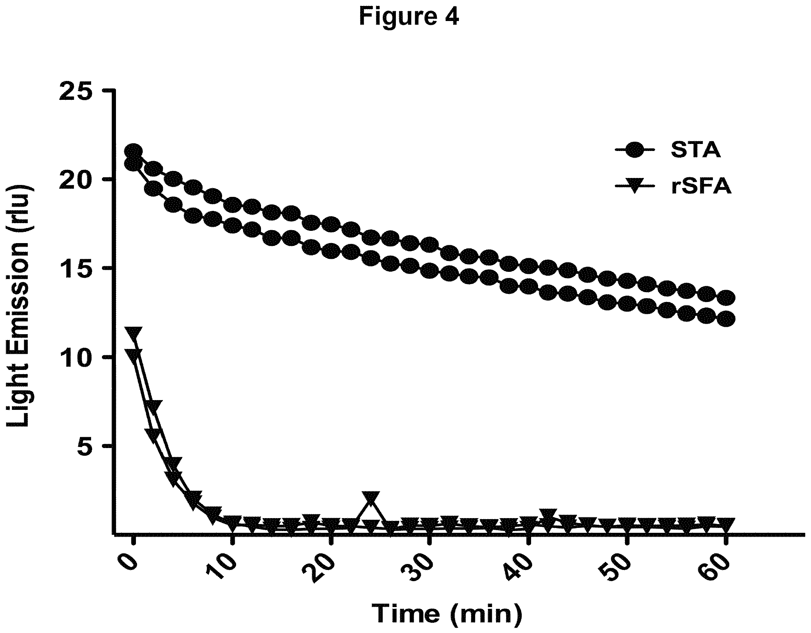

**Figure 4****. Comparison between the activities of recombinant *Shigella flexineri* apyrase (rSFA) and potato apyrase (STA) activities**
   The enzymes, rSFA and STA diluted in 10x in Tris-EDTA Buffer were tested to compare their ATP degradation efficiencies. As can be seen in the figure, rSFA was very efficient in eliminating the ATP, while the commercial STA found to be slower. Even after 60 min of reaction, STA could not eliminate ATP completely, while rSFA eliminated ATP in ^{∼}15 min with a rate constant of 3.87 min⁻¹. Light was measured in the fully automatic 1251 Luminometer (LKB-Wallac, Turku, Finland).

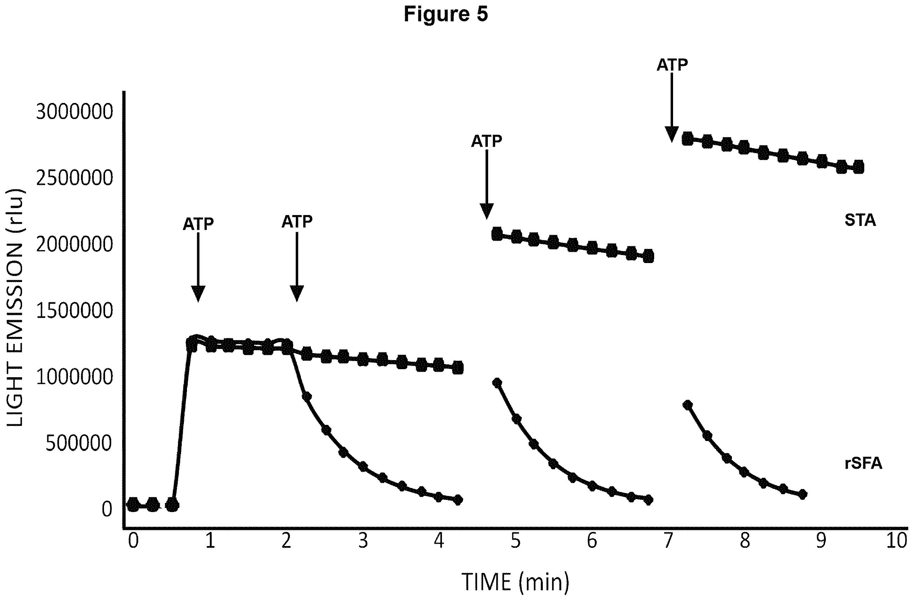

**Figure 5****. Effect of rSFA and STA on ATP and light emission**
   The effect of the addition of recombinant *Shigella flexineri* apyrase (rSFA; diamonds), potato apyrase (STA; squares) and ATP on the light emission from the firefly luciferase reaction. The reaction mixture consisted of 0.2 mL ATP Reagent SL and 0.8 mL Tris-EDTA Buffer (BioThema AB, Sweden). The 1^{st}, 3^{rd} and 4^{th} arrows denote ATP addition and the 2^{nd} arrow apyrase addition. The light was measured every 15 sec except at the additions. Ten µL of 10 µmol/L ATP was added along with 10 µL apyrase. Light was measured in the fully automatic 1251 Luminometer (LKB-Wallac, Turku, Finland).
**Figure 6****. Elimination of extracellular ATP in serum**
   Comparison of ATP elimination with rSFA and STA. In luminescence analysis with 100% crude serum (Fig. 6A), and 10% diluted serum samples (Fig. 6B), rSFA shows very good ATP elimination activity compared to that of STA.
**Figure 7****. Comparison of the activity of rSFA with different commercial STA sources**
   Comparison of three different commercial products of potato apyrase termed STA, A6535 and A6237, to rSFA in buffer. rSFA exhibits superior results.
**Figure 8****. Comparison of the activity of rSFA with different commercial STA sources in 10% serum**
   Comparison of commercial products of potato apyrase like STA, A6535 and A6237 to rSFA in 10% serum. rSFA exhibits superior results.
**Figure 9****. Comparison of the activity of rSFA to commercial STA, in urine**
   Comparison of commercial STA to rSFA in urine, rSFA exhibits superior results. Though both the enzymes showed similar initial activities, rate of action of STA was hindered after 3 min, whereas rSFA continued with ATP-elimination. Even after 4 subsequent injections of 10 µL 10 µmol/LATP, the rSFA showed a complete ATP elimination activity each time in less than 3 min but STA could not digest the ATP even after 20 min.

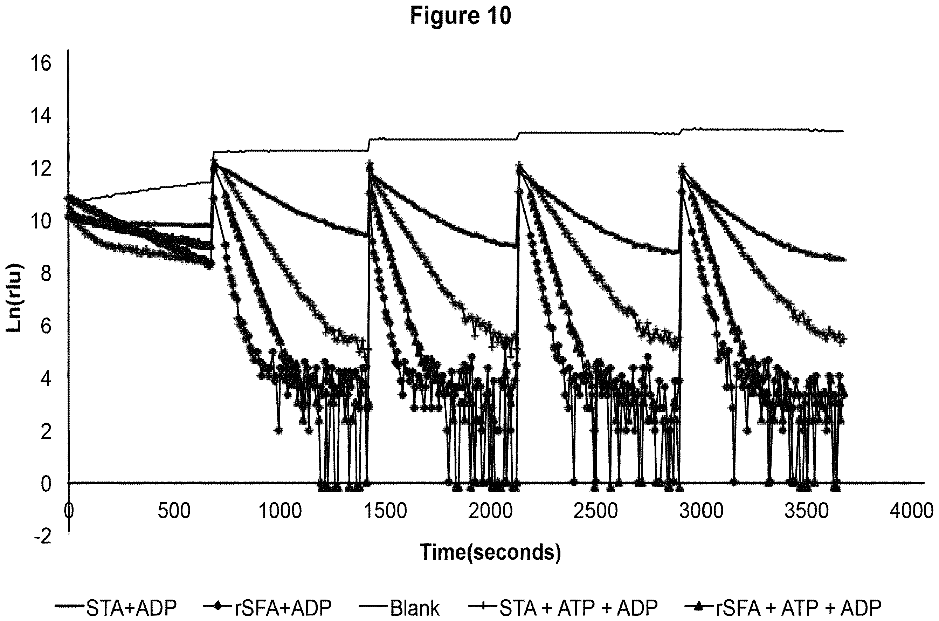

**Figure 10****. Effect of ATP-analogues against STA and rSFA in urine**
   Elimination of ADP at 10 µL 10 µmοl/L and a mixture of ATP and ADP at 10 µL 10 µmol/L were tested in urine samples. The activity of STA was decreased after either by the addition of ADP or in the mixture of ATP and ADP, whereas the activity of rSFA was not affected.

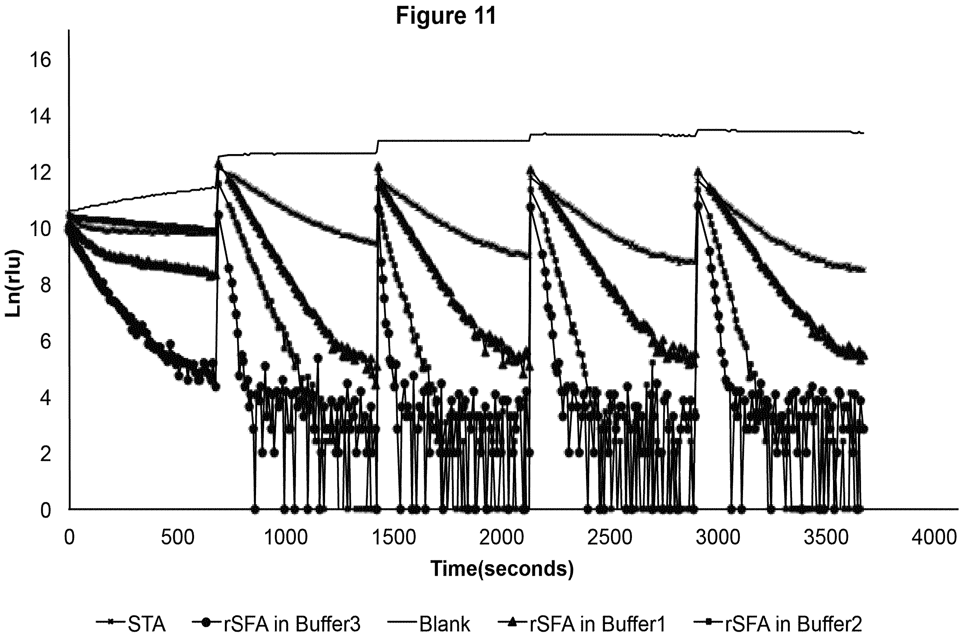

**Figure 11****. Optimization of buffer system to improve the activity of rSFA**
   Equal concentration of rSFA was stored in Buffer 1, Buffer 2 and Buffer 3 for about 10 months and the ATP-elimination assays were performed. The activity of the enzyme was checked intermittently and found that the enzyme used with Buffer 3 showed very good activity compared with other two buffers
**Figure 12****. Development of inhibitors for rSFA**
   Among the different inhibitors tested, 100 mM of MgSO₄ and 1mM of ortho-vanadate in the form of Na₃VO₄ showed inhibitory action against rSFA. The ortho-vanadate at 1 mM did not affect luciferase at all and at the same time, it completely inhibited the activity of rSFA. Magnesium was also able to completely inhibit rSFA at 50 mM, but it also decreased the activity of luciferase.

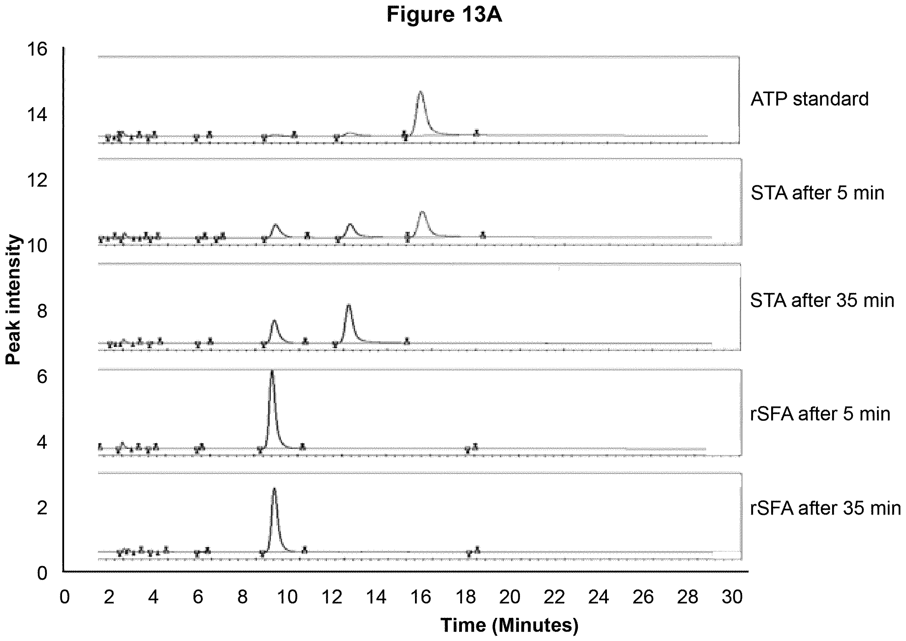

**Figure 13A-E****. Activity of rSFA on ATP, dATP, dTTP, dGTP and dCTP**
   To compare the rSFA activity on nucleotides to STA, samples of ATP, dATP, dTTP, dGTP and dCTP were incubated with the enzyme for 5 min or 35 min and the products detected using high performance liquid chromatography (HPLC). In all instances rSFA resulted in superior elimination of the nucleotide triphosphates and in particular effectively eliminated nucleotide diphophates, which the STA eliminated rather ineffectively.

### DETAILED DESCRIPTION

The inventors have surprisingly found that *Shigella flexneri* apyrase (SFA; known as such from WO1994/012211) is more effective in eliminating contaminating or otherwise unwanted nucleoside triphosphates and nucleoside diphosphates, in particular ATP and/or other ATP-analogues, than the commonly used *Solanum tuberosum* apyrase (STA; potato apyrase).

Table 1 shows a comparison between recombinant *Shigella flexneri* apyrase (rSFA) and *Solanum tuberosum* apyrase (STA; potato apyrase). The amount of STA used was slightly higher in terms of units, and after one minute, the amount of ATP remaining was lower in the STA samples compared to rSFA by nearly a factor of 2. However, at the time point 17 minutes, concentration of ATP in the rSFA treated samples was about 20 times lower compared to STA-treated samples. At 91 minutes the difference was yet more pronounced, the rSFA treated samples having about 45-fold lower concentration than STA-treated samples. It is noteworthy that even after 91 minutes of incubation, the amount of residual ATP was higher in the STA samples compared to the rSFA-samples at 17 minutes.

Without being bound by theory it may be assumed that the greater ability to degrade ATP and its analogues results from one or more of: higher substrate affinity, higher enzyme stability under the assay conditions, lesser inhibition from the reaction end-products. In all ATP-based reactions, presence or accumulation of contaminants or byproducts like ADP, AMP, ATP, dNTP, dNDP, XMP, etc. may interfere with the assay.

Commercial apyrase preparations often contain a mixture of isoenzymes with different relative affinities for ATP, ADP, xMP like tetra- or penta-phosphates, etc. Moreover, the apyrase enzyme activity of different commercial suppliers varies with regard to initial activity and different lots of the same product may show substantially variable ATP-hydrolyzing activities. These variations may be significant obstacles for the development of a robust and sensitive analytical method for analyzing various biological samples.

In this regard, it can be concluded that SFA is much more effective in eliminating traces of above said contaminating ATP and its analogues (or other nucleotides) in a sample that generally hinder the activity and sensitivity of STA. This translates in practical terms that assays for determining the amount of ATP in a sample, where there is or may be contaminating ATP present can achieve greater sensitivity due to better elimination of most of the ATP-analogues, when SFA is used as an apyrase (see Examples 6, 7, 8, and 9). Additionally, in certain circumstances the use of SFA provides for a faster assay by sufficiently eliminating the contaminating ATP in a shorter time. The better elimination of dNTPs (Example 11) also provides for improved sequencing-by-synthesis methods using SFA.

The inventors have also shown that the SFA is not only stable in the reaction conditions but also tolerates freeze-thaw-cycles and storage in solution at refrigerator temperatures, even in crude form, without the addition of stabilizers. The fact that enzyme remains to be active even in high concentrations of EDTA indicates that it does not require any divalent metal ions for its activity, unlike STA. The SFA exhibits very good activity between pH 7.0-7.5, while it remains active between pH 5.0 and 9.5. Therefore the invention provides an opportunity to apply SFA on different biological samples and analytical experiments with a wide range of pH-values. The stability presents a substantial practical advantage, in particular for field laboratories with limited equipment where ATP measurements are performed at times.

Thus, in a first aspect, the present invention relates to a method for reducing the amount of contaminating nucleoside diphosphates and/or nucleoside triphosphates, comprising the steps of
a. providing a sample containing contaminating nucleoside diphosphates and/or nucleoside triphosphates, such as ATP and/or ATP analogues;
b. reducing the amount of the contaminating nucleoside diphosphates and/or nucleoside triphosphates in the sample with an apyrase enzyme, wherein said apyrase enzyme is a *Shigella flexneri* apyrase; and
c. optionally, performing an analysis of the sample, wherein said analysis comprises an assay that would have been affected by the contaminating nucleoside diphosphates and/or nucleoside triphosphates had they not been reduced in step b.

The assay may involve be determination of the amount or concentration of ATP in the sample. By "would have been affected" is meant that the assay is of such character that contaminating nucleoside diphosphates and triphosphates may potentially have an interfering effect. The recitation is not intended to imply that the reduction in step (b) necessarily results in complete elimination of any and all interference. Rather, even less than complete reduction, including partial reduction, substantial reduction, near complete reduction of interference is encompassed.

The contaminating nucleoside triphosphates may comprise deoxyribonucleoside triphosphates (dATP, dTTP, dGTP, dCTP or analogues thereof), and the analysis may be a sequencing-by-synthesis-assay, such as a pyrosequencing reaction.

By "reducing" is meant that the amount is significantly reduced for practical purposes such that the contaminating nucleoside triphosphates or nucleoside diphosphates do not jeopardize the accuracy of the determination in subsequent steps. Preferably, the reduction results in substantial elimination of the contaminating nucleotide to a level of less than 0.01% of the original amount, more preferably to less than 0.001% of the original amount, most preferably to less than 0.0001% of the original amount.

### Methods for determining ATP in a sample

The method of the first aspect may be a method for determining the amount of ATP in a sample, comprising the steps of:
(a) providing a sample containing contaminating ATP and/or ATP analogues including deoxyribonucleoside trphosphates;
b. reducing the amount of contaminating ATP and/or ATP analogues and its analogues in the sample by degradation with an apyrase enzyme;
c. making the ATP to be determined available for determination; and
d. determining the amount of ATP to be measured in the sample,
wherein the apyrase enzyme is a *Shigella flexneri* apyrase.

By "reducing" in the context of ATP determination meant that the amount is significantly reduced for practical purposes such that the contaminating ATP or ATP analogue does not jeopardize the accuracy of the determination in subsequent steps. Preferably, the reduction results in substantial elimination of the contaminating ATP or ATP analogue to a level of less than 0.01% of the original amount, more preferably to less than 0.001% of the original amount, most preferably to less than 0.0001% of the original amount.

The step (b) of making the ATP to be determined available for determination may involve lysis of cells in which the ATP to be determined may be contained, e.g. by heat, denaturing agents, detergents or a combination thereof. The manner of lysis does not matter, as long as the manner used is compatible with the technique used in the determination in step (c). Alternatively, the ATP may be made available by synthesizing it in the sample, e.g. by way of enzymatic conversion of a different chemical species to ATP.

### Methods for determining the amount of ATP present in cells

The method of the first aspect may be a method for determining the amount of ATP present in first population of cells in a sample, comprising the steps of:
(a) providing a sample containing contaminating ATP and/or ATP analogues including deoxyribonucleoside trphosphates;
(b) reducing the amount of contaminating ATP and/or ATP analogues in the sample by degradation with an apyrase enzyme;
(c) liberating the ATP to be determined from the first population of cells; and
(d) determining the amount of liberated ATP;
wherein the apyrase enzyme in step (a) is a *Shigella flexneri* apyrase.

By "liberating" is meant that the ATP is made accessible for the determination in step (c).

The liberation step (b) may involve lysis of the first population of cells, e.g. by heat, denaturing agents, detergents or a combination thereof. The manner of lysis does not matter, as long as the manner used is compatible with the technique used in the determination in step (c).

The first population of cells may comprise bacterial cells. For instance, it may be of interest to determine the number of bacteria present in a clinical sample. Thus, the sample may a biological sample from an animal, such as a human, a canine, a feline, a bovine, an avian or the like. Preferably, the sample is from a human.

The biological sample may for example be a blood sample, a plasma sample, a serum sample, a urine sample or a fecal sample. The sample may be in the form of a swab from a subject, e.g. from skin or a mucous membrane.

The method of the first aspect may comprise the step of adding an apyrase inhibitor after the reduction step. The apyrase inhibitor added may comprise ortho-vanadate. In the context of the present invention, ortho-vanadate is advantageous since it is able to inhibit *Shigella flexneri*-apyrase at a concentration which does not inhibit luciferase. Ortho-vanadate as apyrase inhibitor may be present at a concentration of at least 0.1 mM, preferably 0.2-25 mM, more preferably 0.5-20 mM, most preferably about 1 mM in the sample, Alternatively, magnesium can be used as an apyrase inhibitor. Mg²⁺ may be present at a concentration of at least 25 mM, preferably 30-200 mM, more preferably 30-150 mM in the sample.

In the present methods, it may be desirable to inhibit apyrase activity after the contaminating nucleotides have been eliminated, so that the subsequent analysis of the sample is not detrimentally affected by the apyrase. For instance, the apyrase may be inhibited before the liberation of ATP from the first population of cells discussed above, so that the apyrase does not interfere with the determination of the liberated ATP by eliminating some of it.

At least a fraction of the contaminating ATP (or ATP analogues) may be present in a second population of cells, and the reduction step (a) may be preceded by a step of selective liberation of ATP from the second population of cells. In cases where the second population of cells comprises animal cells and the first population comprises bacterial cells, the animal cells can normally be selectively lysed in much milder conditions than the most types of relevant bacterial cells. For instance, a mild detergent such as Triton X-100 may be used to selectively lyse animal cells in the presence of bacterial cells. See for example US4303752.

The second population of cells may be host cells from the animal from which the biological sample is derived.

The method according to the first aspect may also be applied to non-biological samples, such as cell culture medium, food, beverages, pharmaceuticals, sewage, environmental samples such as swabs from environmental surfaces, drinking water and the like.

The method for determining the amount of ATP present in first population of cells in a sample may additionally comprise step (d) of calculating the number or concentration of viable cells of the first population present in the sample from the amount of ATP determined in step (c). Since the ATP concentration in a cell is fairly constant, the number of cells may be estimated from the amount of ATP determined, e.g. by comparing the reading to a standard curve obtained from a similar type of cell.

### Methods for doing sequencing-by-synthesis, such as pyrosequencing

In general terms, any sequencing-by-synthesis procedure involves sequential addition of a known deoxynucleotide (or an analogue thereof) to a reaction mixture where DNA synthesis will occur dependent on the sequence of the DNA to be sequenced. The addition is followed by determining whether DNA synthesis actually occurred (allowing determination of the sequence), followed by elimination of excess added nucleotide before the next cycle is initiated. It is of importance that the elimination is complete and no intermediate products such as deoxynucleotide diphosphates accumulate, as this would interfere with the subsequent sequencing cycles. The elimination of nucleotides may according to the present disclosure advantageously be performed with SFA, since it exhibits better properties in nucleotide elimination (deoxynucleoside di- and triphosphates) than the commonly used STA (see Example 11). Thus, the method of the first aspect may be a sequencing-by-synthesis procedure, the contaminating nucleotides consist of or comprise excess dNTPs or analogues thereof present after a completed sequencing cycle, and the analysis performed on the sample is a sequence readout.

Pyrosequencing is a well-established DNA sequencing method of the sequencing by synthesis-type, based on the detection of pyrophosphate (PPi) released during DNA synthesis. Briefly, the steps of a typical pyrosequencing protocol can be outlined as follows:
1. A sequencing primer is hybridized to a single stranded DNA template to be sequenced, and incubated with the following enzymes: a DNA polymerase, an ATP sulfurylase, a luciferase and an apyrase, and the following substrates: adenosine 5' phosphosulfate (APS) and luciferin.
2. The first of four deoxynucleotide triphosphates (dNTP) is added to the reaction. It should be noted that deoxyadenosine alpha-thio triphosphate (dATPaS) is used as a substitute for the natural deoxyadenosine triphosphate (dATP) since it is efficiently used by the DNA polymerase, but not recognized by the luciferase. The DNA polymerase then catalyzes the incorporation of the deoxynucleotide triphosphate into the DNA strand, if it is complementary to the base in the template strand. Each incorporation event is accompanied by release of pyrophosphate (PPi) in a quantity equimolar to the amount of incorporated nucleotide.
3. ATP sulfurylase quantitatively converts the PPi to ATP in the presence of adenosine 5' phosphosulfate. The produced ATP drives the luciferase-mediated conversion of luciferin to oxyluciferin that generates visible light in amounts that are proportional to the amount of ATP. The light produced in the luciferase-catalyzed reaction is detected by a detector (such as a CCD-camera). Each light signal is proportional to the number of nucleotides incorporated.
4. Apyrase continuously degrades any unincorporated dNTPs and excess ATP. When degradation is complete, another dNTP can be added to continue with the next cycle in sequencing. Addition of dNTPs is performed one at a time. As the process continues, the complementary DNA strand is built up and the nucleotide sequence can be determined from a graph showing light output over time in relation to the time points of addition of the dNTPs.

Since the commercially available enzymes show batch-to-batch variations in their quality and activity, the applicability of pyrosequencing reaction is limited for sequencing longer oligonucleotides. However, the present invention provides that *Shigella flexneri* apyrase can be used as the apyrase in a pyrosequencing reaction with improved results, since it is more efficient in eliminating deoxynucleoside di- and triphosphates (see Example 11).

Thus, the present invention provides a method of the first aspect, being a method for performing pyrosequencing, and comprising the steps of:
a. performing a pyrosequencing reaction comprising addition of a nucleoside triphosphate;
b. converting the pyrophosphate released in step (a) into ATP via an enzymatic reaction;
c. determining of the amount of ATP formed in step (b);
d. degrading unincorporated nucleoside triphosphate from step (a) and ATP formed in step (b) with an apyrase enzyme;
e. repeating the steps a-d at least once;
wherein the apyrase enzyme is a *Shigella flexneri* apyrase.

### General aspects of the methods

In all of the methods above, the determination of ATP may be performed with a bioluminescent assay. Preferably, the determination of ATP is performed with a bioluminescent assay utilizing luciferin and luciferase. Luciferase may be provided in a composition according to the eigth aspect, to enable inhibition of apyrase concomitant to luciferase addition.

The *Shigella flexneri* apyrase in the methods mentioned above may comprise an amino-acid sequence having sequence identity of 80%, more preferably 85%, even more preferably 90%, yet more preferably 95%, still more preferably 97% to the sequence according to SEQ ID NO: 10. Importantly, only such sequence deviations from the native *Shigella flexneri* apyrase according to SEQ ID NO: 10 that retain the apyrase activity are encompassed in the invention. Most preferably, the *Shigella flexneri* apyrase in the methods above comprises or consists of an amino-acid sequence according to SEQ ID NO: 4. *Shigella flexneri* apyrase may be provided in a composition according to the seventh aspect of the dislosure.

### Apyrase reagents and their production

The second to eighth aspects below are not part of the claimed invention as such, but the disclosure of said aspects may be relevant to the claimed invention.

In a second aspect, herein is disclosed a *Shigella flexneri* apyrase comprising an amino-acid sequence with at least 95%, more preferably 96%, yet more preferably 97%, still more preferably 98% identity, even more preferably 99% sequence identity to the sequence according to SEQ ID NO: 4. Importantly, only such sequence deviations from the *Shigella flexneri* apyrase according to SEQ ID NO: 4 that retain the apyrase activity are encompassed in the invention. Most preferably, the *Shigella flexneri* apyrase of the second aspect comprises or consists of an amino-acid sequence according to SEQ ID NO: 4.

In a third aspect, herein is disclosed to a polynucleotide encoding an apyrase according to second aspect.

In a fourth aspect, herein is disclosed a vector comprising a polynucleotide of the third aspect operably linked to a promoter capable of inducing expression of the polynucleotide, preferably via arabinose induction.

In a fifth aspect, herein is disclosed a host cell comprising a polynucleotide of the third aspect operably linked to a promoter capable of inducing expression of the polynucleotide, or a vector of the fourth aspect.

In a sixth aspect, herein is disclosed a method of producing a recombinant apyrase according the second aspect, comprising the steps of:
culturing host cells according to the fifth aspect in conditions inducing the expression of the polynucleotide; and
recovering the produced recombinant apyrase from the culture.

In a seventh aspect, herein is disclosed a composition comprising a *Shigella flexneri*-apyrase having a pH in the range of 6-9, preferably 7-8, and an ionic strength of at least 300 mM. Preferably, the pH is about 7.5. The buffer may be a HEPES buffer. The ionic strength may be 300-1000 mM, preferably 400-800 mM, more preferably 500-800 mM. The buffer may comprise 400-600 mM NaCl and 20-30 mM MgSO₄. As shown in Example 10, the buffer of the seventh aspect is particularly advantageous in terms of enzyme stability.

In an eighth aspect, herein is disclosed a composition comprising a luciferase and an apyrase inhibitor comprising ortho-vanadate. The advantages of ortho-vanadate are discussed in the context of the first aspect of the present invention. It is advantageous to combine the apyrase inhibitor with the luciferase in a single composition, enabling both to be added in a single action. The concentration of ortho-vanadate should chosen such that the final concentration of ortho-vanadate in the sample after addition of the composition to a reaction mixture being used in a luciferase-based assay is sufficient to inhibit SFA. The concentration of ortho-vanadate may be at least 0.2 mM, preferably at least 1 mM, more preferably 1-3000 mM, most preferably 2-200 mM. The amount of luciferase activity should be sufficient for a luciferase-based assay after addition of the composition to a reaction mixture.

### Uses of SFA apyrase

In a ninth aspect, the present invention relates to a use of a *Shigella flexneri* apyrase for degrading contaminating nucleotides (nucleotide triphosphates and/or nucleotide diphosphates) in an analytical method. The contaminating nucleotides may be ATP and/or ATP analogues, and the analytical method may be an assay for measuring ATP. The assay for measuring ATP may be a bioluminescent assay.

In an tenth aspect, the present invention relates to a use of a *Shigella flexneri* apyrase in a DNA sequencing reaction, preferably a sequencing-by-synthesis reactions, most preferably a pyrosequencing method.

The apyrase in the ninth or the tenth aspects may comprise an amino-acid sequence having sequence identity of 80%, more preferably 85%, even more preferably 90%, yet more preferably 95%, still more preferably 97% to the sequence according to SEQ ID NO: 10. Importantly, only such sequence deviations from the native *Shigella flexneri* apyrase according to SEQ ID NO: 10 that retain the apyrase activity are encompassed in the invention. More preferably, the apyrase in the ninth and the tenth aspects may be an apyrase according to the second aspect.

The arrangement of the present disclosure into sections with headings and subheadings is merely to improve legibility and is not to be interpreted limiting in any way, in particular, the division does not in any way preclude or limit combining features under different headings and subheadings with each other.

The following examples are not to be construed as limiting of the invention.

### EXAMPLES

For experimental details concerning the examples below, the reader is directed to a separate section titled Materials and Methods.

### Example 1: Production and colorimetric evaluation of recombinant Shigella flexneri apyrase (SFA)

The SFA was produced and purified as described in the materials and methods section. The purified enzyme fraction was examined using a colorimetric assay to identify the presence of bacterial apyrase. As can be seen in Fig. 1, the colorimetric assay clearly shows the color difference that directly corresponds to expression levels of apyrase enzyme expression, thus revealing its cellular location. In all of the cases, a clear difference in the color can be seen between non-induced and induced cultures in comparison with the color of the blank sample.

As shown in Fig. 2, a clear correlation can be drawn between the colorimetric results and apyrase expression profiles of the clones and their localization. The enzyme, rSFA was constantly overexpressed in induced cultures at 25 kDa, especially in whole cell lysates and soluble fractions, but not in membrane fractions, thereby indicating its location for the further purification procedures. The induced cultures revealed an expression of ^{∼}10 times more enzyme compared to that of un-induced cells, which demonstrated the efficiency of the constructed clone. This SDS-PAgel also shows the purity of the apyrase production and this purified fraction was used for all the experiments of this study.

### Example 2: Sequence analysis between rSFA and STA

Once the protein expression was confirmed by gel electrophoresis and colorimetric assay, the rSFA gene product was sequenced to identify the nucleotide sequence in order to compare with the reference sequence WP_010921592.1 obtained from the NCBI database (Figure 3A). As can be seen in the figure, the cloned oligonucleotide fragment of SFA containing Apyrase-6xHis (rSFA) and the expressed sequence (pBL21-Apyrase-6xHis) sequences fell in the desired range (see Figure 3A). As can be seen in Figure 3B, the rSFA differs from the reference SFA only at N- and C-termini.

### Example 3: rSFA is more efficient in ATP-degradation compared to STA and is stable

The measurements of the decay of the light emission from the firefly luciferase reaction (Fig. 4) described the ATP-degradation activity between the two apyrases, rSFA and STA. The rSFA reveals a clear elimination of ATP and its analogues in ^{∼}10 min compared to that of STA. In the other experiment, as can be seen in Figure 5, luminescence was increased upon the addition of ATP in both cases, while, addition of apyrase resulted in a decay of the light because of ATP depletion. Deliberate addition of extra ATP during the reaction revealed a similar ATP depletion trend. However, the rSFA, exhibited more rapid ATP-depletion activity than did the commercially available STA. When ATP as well as the apyrase preparations was diluted 10-fold, the ATP depletion was reduced accordingly.

The rSFA samples were kept at 4°C, then frozen and freeze-thawed to check its stability. An effort was made to see if the rSFA could be used to prepare a reagent for the depletion of extraneous ATP in biological samples.

**Table 1. ATP-degradation activity comparison between recombinant Shigella flexneri apyrase and commercially available potato apyrase procured from Sigma-Aldrich**

| **Type of apyrase** | **Initial activity (Units/mg protein)** | **Remaining ATP activity** | | |
|---|---|---|---|---|
| | | after 1 min | after 17 min | after 91 min |
| Potato apyrase (STA) | 4,12 | 1,1876% | 0,0367% | 0,0228% |
| Recombinant *Shigella* apyrase (rSFA)* | 3,39 | 2,6953% | 0,0019% | 0,0005% |
| Recombinant *Shigella* apyrase (rSFA)** | 3,58 | 2,1855% | 0,0019% | 0,0004% |
| *Stored frozen before experiment | | | | |
| **Stored in refrigerator before experiment | | | | |

Furthermore the remaining ATP was much lower after longer incubation times as shown in the Table 1. This indicates that the SFA, is more sensitive and efficient than STA in ATP depletion, as ADP accumulates in the reaction mixture and so hinders the reaction efficiency of the latter.

### Example 4: Demonstration of extracellular ATP and its analogues in serum

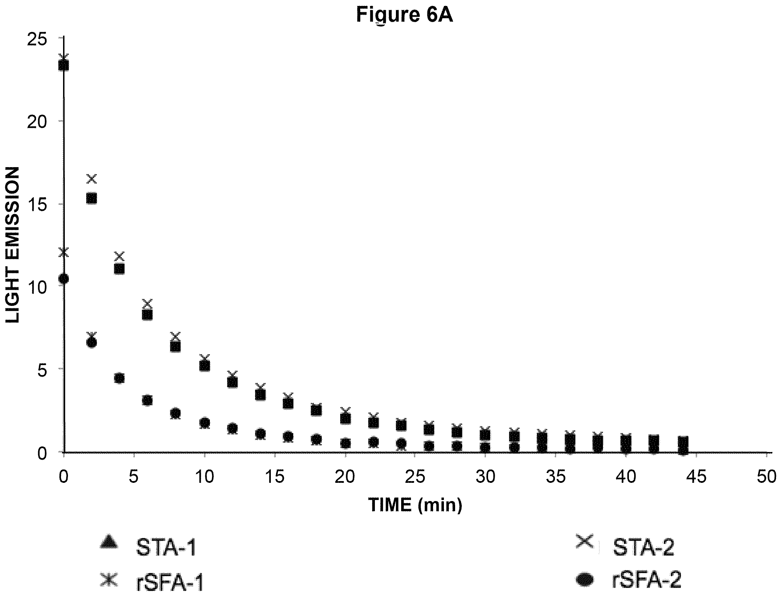

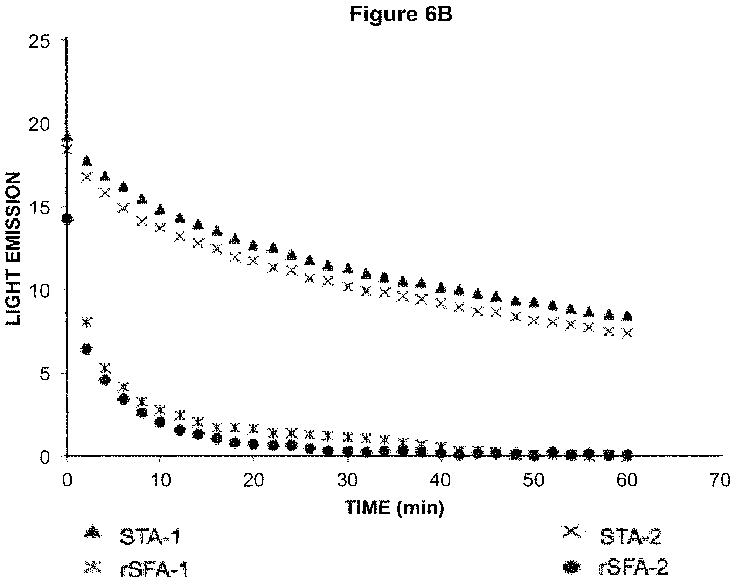

Considering the advantages of rSFA from the above experiments, we checked its efficiency removing extracellular ATP in serum samples. In the first experiment (Fig. 6A), rSFA revealed complete hydrolysis of ATP and its analogues ^{∼}17 min, whereas STA took ^{∼}45min, against 100% crude serum. The linearity was reproduced (Fig. 6B), even when the serum was diluted to 10%. Hence rSFA is superior to that of STA in eliminating ATP in biological samples like serum.

**Table 2: The effect of serum on ATP depletion rate in crude and diluted serum**

| | No serum (Negative control) | Undiluted serum (100% crude serum) | 10% Diluted serum |
|---|---|---|---|
| potato apyrase | 3,52 | 0,55355219 | 2,42904382 |
| recombinant apyrase | 3,98 | 1,81641444 | 1,77443406 |

### Example 5: Improved method for pyrosequencing

Since the commercially available enzymes show batch-to-batch variations in its quality and activity and inability to hydrolyze excess of ATP and its analogues, it decreases the efficiency and limits the applicability of pyrosequencing reaction to read long sequences. This can be addressed using rSFA because it shows better quality and efficiency compared to STA. See also Example 11.

### Example 6: rSFA is superior to STA in an ATP bioluminescent assay, in buffer

The time course of activities, including initial and end-point activities of three commercial products of potato apyrase termed STA, A6535 and A6237 were compared with rSFA in a bioluminescent ATP assay. The enzymes were diluted in a buffer, so no sample interference should be present and the results represent an ideal scenario. The measurement was light output from a luciferase, and the desired result is as fast and complete as possible elimination of ATP by the apyrase.

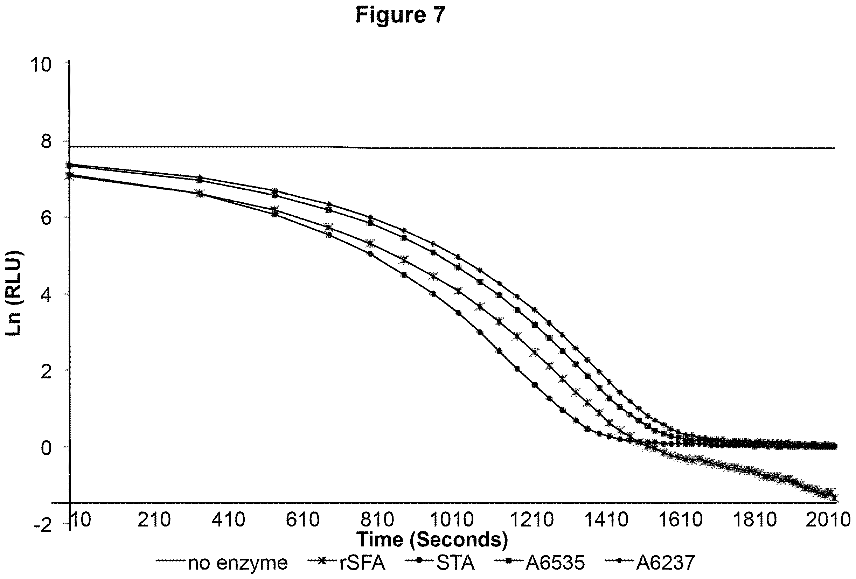

As shown in Figure 7, the STA variant A6237 showed better activity compared to the other commercial STA sources. Nevertheless, all the commercial STA variants were saturated after 120 s and remained ineffective in further reducing the ATP concentration thereafter. In contrast, rSFA was active in eliminating the ATP-analogues and its by-products even after 300 s and is capable of a more complete elimination of ATP. Note the log scale in the graph, indicating at least 10-fold improvement.

### Example 7: rSFA is superior to STA in an ATP bioluminescent assay, in 10% serum

An experiment similar to Example 8, but with 10% serum present in the assay mixture was undertaken to test utility of rSFA in samples containing serum, such as blood samples.

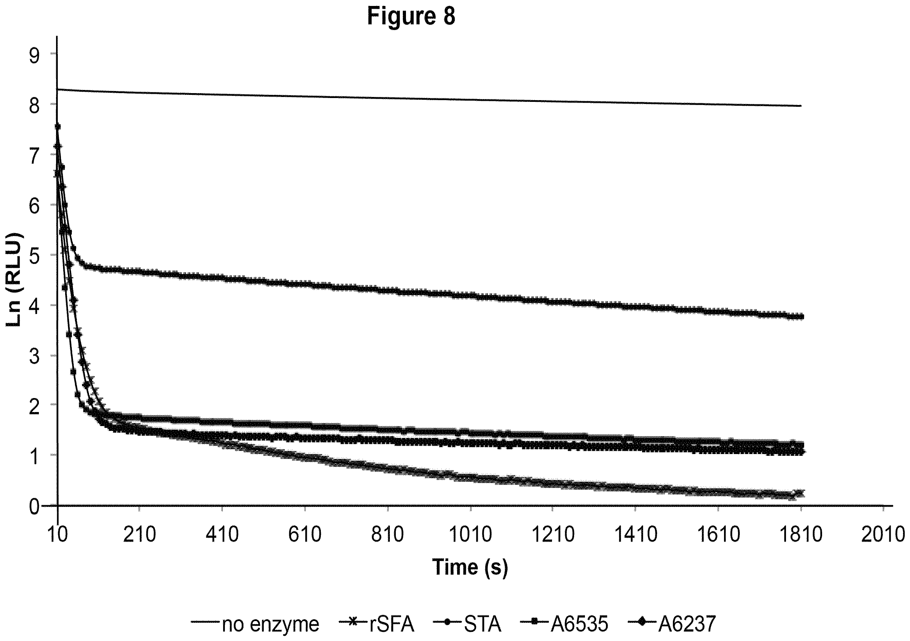

As depicted in Figure 8, activity of the commercial STA variants (STA, A6535 and A6237) did not show further ATP elimination after 6 min but rSFA continued to cleave ATP-analogues and its by-products even after 30 min. Presence of extracellular ATP could potentially inhibit the activity of STA, whereas rSFA remains active in removing extracellular ATP from 10% serum. Thus, rSFA is superior to STA in the analysis of blood samples.

### Example 8: rSFA is superior to STA in an ATP bioluminescent assay, in urine

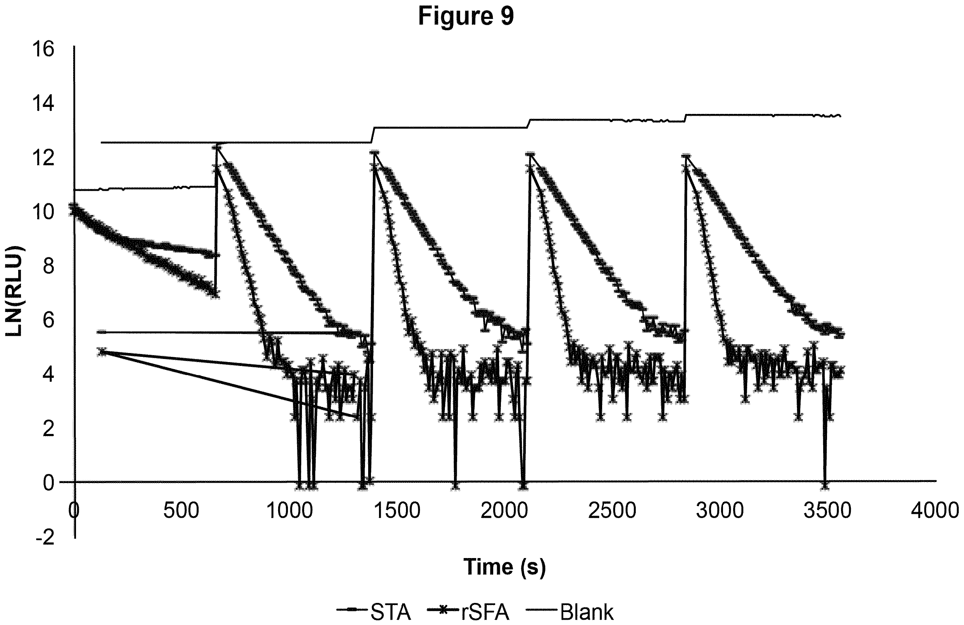

Considering the complications due to contaminants in urine, the inventors tested the ATP-elimination activities of STA and rSFA for 60 min in an urine sample. Though both the enzymes showed similar initial activities, rate of action of STA was hindered after 3 min, whereas rSFA continued with ATP-elimination. Even after 4 subsequent injections of 10 µL 10 µmol/L ATP, the rSFA showed a complete ATP elimination activity each time in less than 3 min but STA could not digest the ATP even after 20 min (Figure 9).

### Example 9: Effect of ATP-analogues on the activities STA and rSFA

Hydrolysis of ATP, ADP and their mixtures in urine was tested with STA and rSFA. Fig. 10 shows that the activity of STA was considerably decreased either against only ADP 10 µmol/L or when a mixture of ADP and ATP was supplemented at 10 µmol/L concentrations. In contrast, there was no substantial difference in the activity with rSFA. In both the cases, the STA's phosphate removal activity from ATP-analogues was significantly lower compared to that of rSFA.

### Example 10: Optimization of buffer system to improve the activity of rSFA

Selection and optimization of a correct buffer system is an important aspect of enzyme stability. The inventors have developed three buffer systems to optimize the stability of rSFA. Equal concentration of rSFA was stored in Buffer 1, Buffer 2 and Buffer 3 for about 10 months at +4°C and the bioluminescent ATP-elimination assays were performed. The activity of the enzyme was assayed and it was found that the enzyme used with Buffer 3 showed particularly good activity compared with other two buffers (Figure 11).

### Example 11: Activity of rSFA on ATP, dATP, dTTP, dGTP and dCTP

To compare rSFA activity on nucleotides to that of STA, ATP, dATP, dTTP, dGTP and dCTP were incubated with the enzymes (same initial activity) for 5 min and 35 min and the resulting products were detected using high performance liquid chromatography (HPLC).

As a starting point, chromatographic analysis of ATP (Fig. 13A) revealed that it is only partially cleaved to ADP and AMP in 5 min by STA, and all of ATP was completely converted into ADP and AMP after 35 min. In contrast, rSFA fully converted all ATP into AMP in less than 5 min with no detectable ADP remaining.

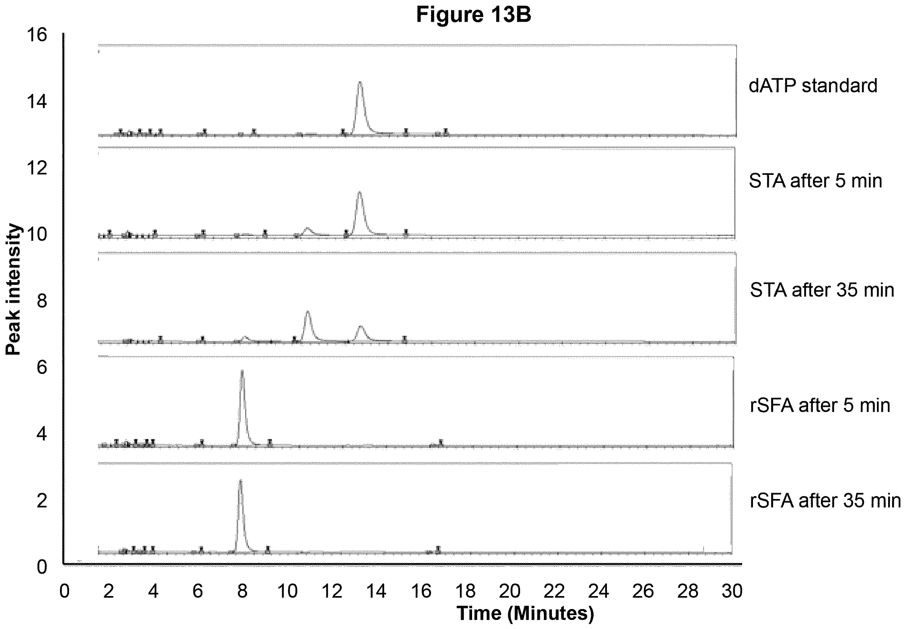

Fig. 13B shows that more than 25% of dATP was remaining after 35 min of incubation with STA (product mainly dADP, and some dAMP), whereas rSFA fully converted the dATP into dAMP in less than 5min.

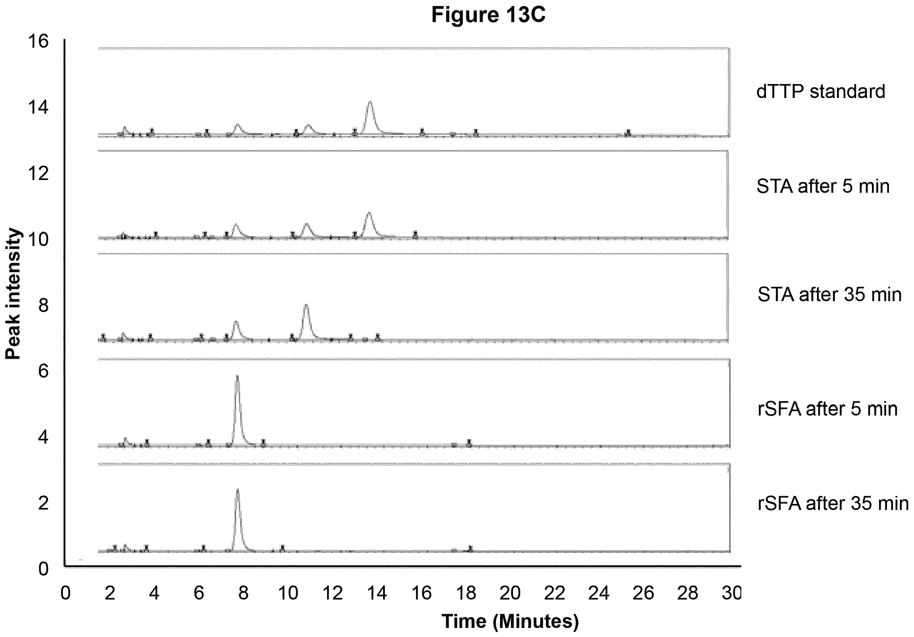

Similarly, dTTP was converted in to TDP and TMP in 35 min by STA, whereas rSFA fully converted dTTP it into TMP in less than 5 min (Fig. 13C).

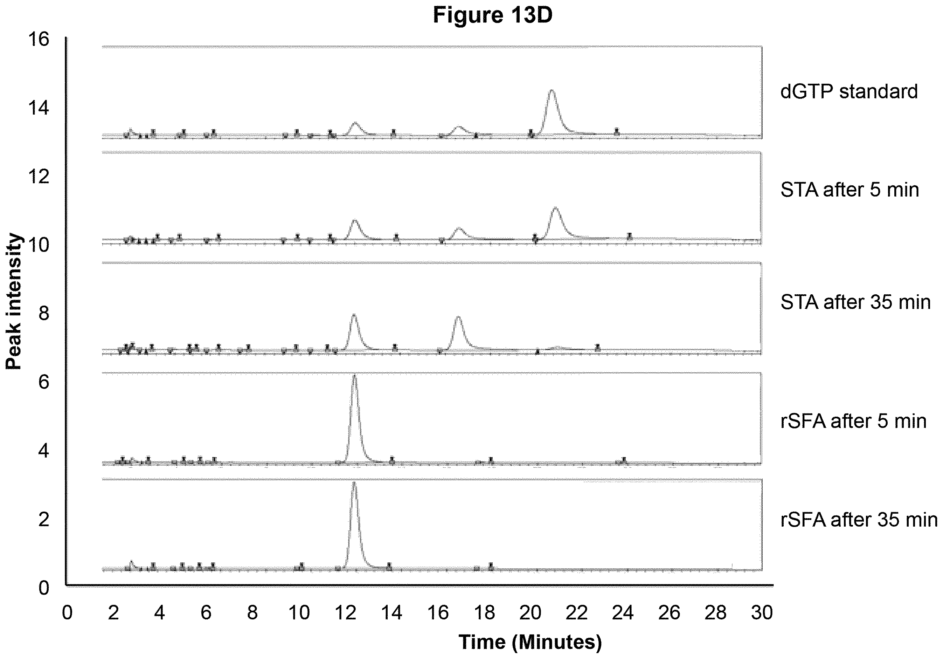

Similar results were observed with dGTP (Fig. 13D).

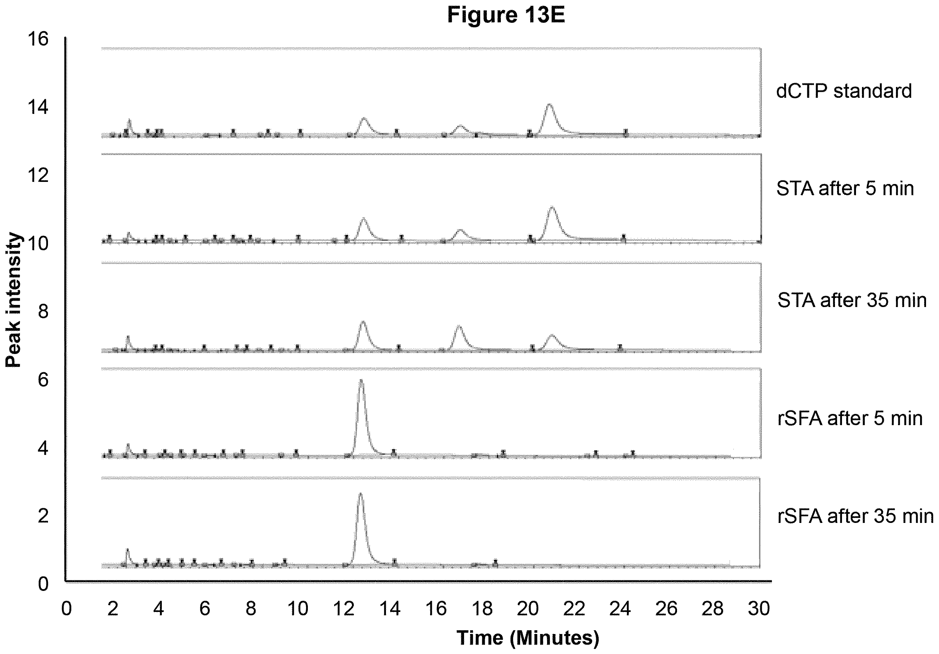

However, as can been in Fig. 13E, the STA could convert only half of the dCTP into CDP and CMP after 35 min,whereas rSFA completely converted the dCTP into CMP in less than 5 min. Therefore, rSFA is at least 7 times more potent in eliminating dCTP than STA.

The above results indicate that SFA is superior to STA for eliminating dNTPs and dNDPs, which is in particular relevant for use in a sequencing-by-synthesis method (such as pyrosequencing), since the accumulation of dNTP and/or dNDPs interfere with subsequent sequencing cycles.

### Example 12: rSFA can be inhibited by ortho-vanadate without interferering with luciferase activity

The activity of rSFA may in some cases need to be stopped in order to measure the intracellular concentration of ATP. Among the different inhibitors tested, 100 mM of MgSO₄ and 1mM of ortho-vanadate in the form of Na₃VO₄, where only the former affected the activity of luciferase (Figure 12). The ortho-vanadate did not affect luciferase and at the same time, it completely inhibited the activity of rSFA, making it ideal for inhibiting apyrase in an assay involving luciferase.

### Materials and Methods

### Cloning, sequencing and production of Shigella apyrase

The pRSETB plasmid bearing *Shigella flexneri* 2a *apy* gene in *E. coli* GJ1158 strain (Bhandari and Gowrishankar J Bacteriol. 1997 Jul;179(13):4403-6) was obtained from Sankaran, Centre for Biotechnology, Anna University, India. Since the GJ1158 construct was not stable, the native *apy* gene was excised and amplified (using apy 3 and apy 4 primers; Table 3), which was further purified by PCR gel purification Kit (Fermentas). The purified DNA template was introduced with two-endonuclease restrictase sites for *NdeI and BsiWI* to replace natural stop codon by 6-Histidine-coding region using the primers Apyr-6His-BsiWI and Apyr-5-Ndel (Table 2) and transformed in *E. coli* TOP10 (Invitrogen) according to the manufacturer's instructions with minor modifications. Transformants were grown on agar plates and the positive pBL-Apyrase-6His clones were selected by *Nhel* hydrolysis and size verification of restriction fragments on agarose gels, followed by sequence verification at Karolinska Institute's sequencing facility, Stockholm. Identified sequences of pBL-Apyrase-6His constructs were compared with *Shigella flexneri* 2a apyrase (GeneBank accession: U04539) to find out the integrity of cloning sites, Apyrase-6His coding sequence and replacement of the stop codon by 6-His tag. After sequence confirmation, the plasmid of pBL-Apyrase-6His clone (SEQ ID NO: 3) was transformed in *E. coli* BL21-A1 for the protein expression. The amino-acid sequence encoded by the plasmid is shown in SEQ ID NO: 4.

**Table 3. List of oligonucleotides used**

| **Name** | **Oligonucleotide sequence (5' → 3')** | **SEQ ID** NO |
|---|---|---|
| **Native apyrase primer1** | CGCGGATCCCTGAAGGCAGAAGGTTTTC | SEQ ID NO: 5 |
| **Native apyrase primer2** | CCCAAGCTTTTATGGGGTCAGTTCATT | SEQ ID NO: 6 |
| Apyr-6His-BsiWI | | SEQ ID NO: 7 |
| Apyr-5-Ndel | GATATACATATGAAAACCAAAAACTTTC | SEQ ID NO: 8 |

### Large-scale production of pure Shigella apyrase

**Preliminary evaluation using Ni-NTA magnetic beads:** About 1.3 ml of bacterial culture was pelleted at 13,000 rpm and 300 µl of extraction buffer (1 mM PMSF, 1 mg/ml lysozyme and 0.05% Triton X-100 in 20 mM Tris-HCl, pH 8.0) to prepare cell lysates. After 30 min, 30 µl of equilibrated Ni-NTA magnetic beads were added and kept on mild agitation to facilitate the binding of His-tagged protein to the beads. Supernatant containing the soluble proteins was sampled and the beads were washed with 300 µl of washing buffer (300 mM NaCl, 20 mM imidazole, 8% glycerol and 0.2% Triton X-100 in 10 mM KP, pH 7.8). After rinsing the beads with PBS for 10 min, they were boiled for 3 min with 12 ul of SDS to elute pure recombinant protein. The protein profiles of cell lysates, soluble proteins and pure protein fractions were resolved on 12% SDS-PA gels at 30 mA using Tris-glycine (25 mM Tris-HCl, 0.1% w/v SDS, pH 8.3). After confirmation, apyrase production was scaled-up to large scales up to 2 L.

**Large-scale purification by metal affinity chromatography:** The *apy* gene (SFA) bearing *E. coli* BL21-A1 strain containing pBL-Apyrase-6His was cultured in 2 L of LB medium and induced at OD 0.7. Cells were harvested by centrifuging at 4000 rpm after 4h of growth and after a saline wash they were lysed by French press. The 6-histidine-tagged SFA was purified by metal affinity (IMAC) (HisTrap FF, GE-Healthcare, Sweden) using a buffer (20 mM Hepes, 500 mM NaCl, 10% glycerol, 20 mM imidazole, pH 7.5) and after washing with a wash buffer containing 50 mM imidazole, the enzyme was eluted with elution buffer containing 350 mM imidazole. The buffer was exchanged with buffer containing 25mM MgSO₄, 5% BSA and the protein sample was concentrated using an ultrafiltration device with a 10 kDa cut-off semi-permeable membrane (Vivaspin Turbo, Sartorius).

### Colorimetric evaluation of apyrase activity

The enzyme activity was evaluated by calorimetrically, as described by Sankaran et al. (Diagn Microbiol Infect Dis. 2009 Mar;63(3):243-50) with minor modifications. The colorimetric reaction was performed by mixing equal volumes of assay reagent (6M sodium pyrophosphate and 40mM EDTA; pH 7.5) and a color reagent (5% w/v acidic ammonium molybdate and 1 % w/v ferrous ammonium sulfate) in the presence of apyrase enzyme (test sample) to produce colorimetric signals, which are proportional to the concentration of enzyme present in the test sample. Principally, the assay uses inorganic pyrophosphate as a substrate for the enzyme, while EDTA acts both as a buffer and a metal chelator to suppress the other contaminating phosphatase and pyrophosphatase activities. The released phosphate is measured in the presence of pyrophosphate using acidic ammonium molybdate and the color was measured at 695 nm in a multi-mode microplate reader (SpectraMax® M5, Molecular Devices, LLC, USA).

### Sensitivity tests by luminescent experiments

Cuvettes contained 100 µL sample in a total volume of 1 mL containing ATP Reagent SL (BioThema AB, Sweden) in 0.07 mol/L Tris- EDTA Buffer and the reaction was started by injection of 10 µL 10 µmol/L ATP Standard. The initial apyrase activity was determined as described by Lundin and coworkers (Analytical Biochemistry 75, 611-620 (1976) and Methods in Enzymology vol 305, 346-370 (2000)) with some modifications. Bioluminescence was measured at every 10 s during 1 min, in the fully automatic 1251 Luminometer (LKB-Wallac, Turku, Finland). To this, 10 µl of 10 µmol/L ATP was added to determine the background ground luminescence. The temperature control was set to 25°C and bioluminescence was measured from several cuvettes in parallel during 30 or 60 minutes and compared to residual ATP after various incubation times using rSFA and STA. This was repeated with rSFA and STA and a graph was plotted by keeping the ATP-degradation rate as internal control to determine the ATP-degradation efficiency of both of the enzyme sources.

### Bioinformatic analysis of different apyrases (ATP-diphosphohydrolase)

The DNA sequence of *Shigella flexneri* 2a apyrase (GI 532975) (SEQ ID NO: 2) and of the translated potato apyrase (Translated Gene ID 1025774 of *Solanum tuberosum* ATP-diphosphohydrolase NM_008XM_004845) (SEQ ID NO: 1) sequences were obtained from the NCBI nucleotide database. These nucleotides were analyzed using online tools indicated above for the homology search, multiple gene alignments and their translations to protein sequences.

### Optimization of buffer system

Equal concentration of rSFA was stored in Buffer 1(20mM Tris, 1 mM EDTA, 1% BSA, 25 mM MgSO₄, pH 7.5), Buffer 2 (20 mM Tris, 25 mM MgSO₄, 2.5% BSA, pH 7.5) and Buffer 3 (20 mM HEPES, 500 mM NaCl, 25 mM MgSO₄, pH 7.5) for about 10 months at 4°C. Bioluminescence activity assay was performed as described below to detect the activity of rSFA after storage.

### Inhibitor development

After checking the rSFA inhibitory activity in buffer, it was verified with urine, where 10 µl were added with 80 µl of cell lysate reagent and 10 µmol/L of ATP. About 100 mM of MgSO₄ and 1 mM of Na₃VO₄ were added in separate tubes and incubated for 10 min before measuring the light emission using firefly luciferase.

### Bioluminescence assay

Light emission from the firefly-luciferase reaction was measured at 25 °C in a 1251 Luminometer (LKB-Wallac; Finland) using the ATP Kit SL (BioThema AB, Sweden) according to the manufacture's instruction (in buffer experiment) or adding concentrated luciferase (in serum/urine experiments). Initially luciferase reaction follows first-order kinetics and the ATP Reagent SL in this kit produces light intensity proportional to the ATP concentration. A plot of the natural logarithm of the light intensity versus time yields a straight line with the slope representing the rate constant of the reaction. Starting concentration of STA, A6535, A6237 (Sigma-Aldrich) was adjusted similar to rSFA. BL-reaction was started automatically upon addition of 0.2µmol/L ATP, and the light intensity was measured every 10 second for 10 minutes (in buffer experiment) or 30 minutes (in serum and urine experiments), in the reaction volume of 0.5mL.

### High performance liquid chromatography (HPLC) analysis of nucleosides/nucleotides

The dNTP Standard preparation was prepared to contain final concentration of 0.1mM enzyme. Nucleosides ATP/dATP/dCTP/dGTP/dTTP were suspended in Tris - EDTA buffer and diluted 10 times with the mobile phase (70% acetonitrile, 30% ammonium acetate pH 5.35). The above-prepared nucleosides were individually incubated with equal amounts of rSFA and STA for 35 min at pH 7.75 and 5µl of the mixture was injected into HPLC instrument (SeQuant ZIC®-pHILIC column, Merck Millipore) at a flow rate of 0.7mL/min using a pump (Jasco PU-2089 Plus). Peaks were detected at 254nm by UV-2075 Plus detector and the chromatographic separation was achieved using isocratic elution. Analysis was performed using Chrompass software, where each nucleotide/nucleoside was identified by comparison with retention time of the respective standards.

## Claims

1. A method for reducing the amount of contaminating nucleoside diphosphates and/or nucleoside triphosphates, comprising the steps of
a. providing a sample containing contaminating nucleoside diphosphates and/or nucleoside triphosphates, such as ATP and/or ATP analogues including deoxyribonucleoside triphosphates;
b. reducing the amount of the contaminating nucleoside diphosphates and/or nucleoside triphosphates in the sample with an apyrase enzyme, wherein said apyrase enzyme is a *Shigella flexneri* apyrase; and
c. performing an analysis of the sample, wherein said analysis comprises an assay that would have been affected by the contaminating nucleoside diphosphates and/or nucleoside triphosphates had they not been reduced in step b.

2. A method for determining the amount of ATP in a sample, comprising the steps of:
a. providing a sample containing contaminating ATP and/or ATP analogues including deoxyribonucleoside triphosphates;
b. reducing the amount of contaminating ATP and/or ATP analogues in the sample by degradation with an apyrase enzyme;
c. making the ATP to be determined available for determination; and
d. determining the amount of ATP to be determined in the sample, wherein the apyrase enzyme is a *Shigella flexneri* apyrase.

3. A method for determining the amount of ATP present in a first population of cells in a sample, comprising the steps of:
a. providing a sample containing contaminating ATP and/or ATP analogues including deoxyribonucleoside triphosphates;
b. reducing the amount of contaminating ATP and/or ATP analogues in the sample by degradation with an apyrase enzyme;
c. liberating the ATP to be determined from the first population of cells; and
d. determining the amount of liberated ATP; wherein the apyrase enzyme in step (b) is a *Shigella flexneri* apyrase.

4. The method according to claim 3, wherein the liberation step (b) involves lysis of the first population of cells; the first population of cells comprises bacterial cells; and wherein the sample is a biological sample from an animal or a human.

5. The method according to any of claims 1-4, wherein the sample is a blood sample, a plasma sample, a serum sample, a urine sample, a fecal sample, or a swab from a patient.

6. The method according to any of claims 2-5, wherein at least a fraction of the contaminating ATP is present in a second population of cells, and the reduction step (b) is preceded by a step of selective liberation of ATP from the second population of cells, wherein the second population of cells are host cells from an animal from which the sample is derived.

7. The method according to any of claims 1-6, where the method comprises the step of adding an apyrase inhibitor after the reduction step.

8. The method according to any of claims 1-3 or 7, wherein the sample is a cell culture medium sample, food sample, a beverage sample, a pharmaceutical sample, a sewage sample, an environmental sample such as a swab from a surface, or a drinking water sample.

9. The method according to claim 1, wherein the method comprises a sequencing-by-synthesis procedure, the contaminating nucleotides comprise excess dNTPs or analogues thereof present after a completed sequencing cycle, and the analysis performed on the sample is a sequence readout.

10. A method for doing pyrosequencing comprising the steps of:
a. performing a pyrosequencing reaction comprising addition of a nucleoside triphosphate;
b. converting the pyrophosphate released in step (a) into ATP via an enzymatic reaction;
c. determining the amount of ATP formed in step (b);
d. degrading unincorporated nucleoside triphosphate from step (a) and ATP formed in step (b) with an apyrase enzyme;
e. repeating the steps a-d at least once;
wherein the apyrase enzyme is a *Shigella flexneri* apyrase.

11. The method according to any of the preceding claims, wherein the *Shigella flexneri* apyrase comprises an amino-acid sequence with at least 80% sequence identity to SEQ ID NO: 10.

12. The method according to any of the preceding claims, wherein *Shigella flexneri* apyrase is provided in a buffer having a pH in the range of 6-9, and an ionic strength of at least 300 mM.

13. A use of a *Shigella flexneri* apyrase for degrading contaminating nucleoside triphosphates or nucleoside disphosphates in an analytical method.

14. The use according to claim 13, wherein the contaminating nucleosides comprise contaminating ATP and/or ATP analogues, and the analytical method comprises an assay for measuring ATP.

15. A use of a *Shigella flexneri* apyrase in a sequencing by synthesis assay, preferably a pyrosequencing assay.

## Patentansprüche

1. Verfahren zum Reduzieren der Menge von verunreinigenden Nucleosiddiphosphaten und/oder Nucleosidtriphosphaten, umfassend folgende Schritte
a. Bereitstellen einer Probe, die verunreinigende Nucleosiddiphosphate und/oder Nucleosidtriphosphate wie ATP und/oder ATP-Analoga einschließlich Desoxyribonucleosidtriphosphate enthält;
b. Reduzieren der Menge der verunreinigenden Nucleosiddiphosphate und/oder Nucleosidtriphosphate in der Probe mit einem Enzym Apyrase, wobei das Enzym Apyrase eine Apyrase von *Shigella flexneri* ist; und
c. Durchführen einer Analyse der Probe, wobei die Analyse einen Assay umfasst, der durch die verunreinigenden Nucleosiddiphosphate und/oder Nucleosidtriphosphate beeinträchtigt worden wäre, wenn sie in Schritt b. nicht reduziert worden wären.

2. Verfahren zum Bestimmen der Menge von ATP in einer Probe, das folgende Schritte umfasst:
a. Bereitstellen einer Probe, die verunreinigendes ATP und/oder verunreinigende ATP-Analoga einschließlich Desoxyribonucleosidtriphosphate enthält;
b. Reduzieren der Menge von verunreinigendem ATP und/oder verunreinigenden ATP-Analoga in der Probe durch Abbau mit einem Enzym Apyrase;
c. Verfügbarmachen des zu bestimmenden ATP für die Bestimmung; und
d. Bestimmen der Menge von zu bestimmendem ATP in der Probe, wobei das Enzym Apyrase eine Apyrase von *Shigella flexneri* ist.

3. Verfahren zum Bestimmen der Menge von ATP, die in einer ersten Zellpopulation in einer Probe vorhanden ist, das folgende Schritte umfasst:
a. Bereitstellen einer Probe, die verunreinigendes ATP und/oder verunreinigende ATP-Analoga einschließlich Desoxyribonucleosidtriphosphate enthält;
b. Reduzieren der Menge von verunreinigendem ATP und/oder verunreinigenden ATP-Analoga in der Probe durch Abbau mit einem Enzym Apyrase;
c. Freisetzen des zu bestimmenden ATP aus der ersten Zellpopulation; und
d. Bestimmen der Menge von freigesetztem ATP; wobei das Enzym Apyrase in Schritt (b) eine Apyrase von *Shigella flexneri* ist.

4. Verfahren nach Anspruch 3, wobei der Freisetzungsschritt (b) eine Lyse der ersten Zellpopulation umfasst; die erste Zellpopulation Bakterienzellen umfasst und wobei die Probe eine biologische Probe von einem Tier oder Menschen ist.

5. Verfahren nach einem der Ansprüche 1 bis 4, wobei die Probe eine Blutprobe, eine Plasmaprobe, eine Serumprobe, eine Urinprobe, eine Stuhlprobe oder ein Abstrich von einem Patienten ist.

6. Verfahren nach einem der Ansprüche 2 bis 5, wobei zumindest ein Anteil des verunreinigenden ATP in einer zweiten Zellpopulation vorliegt, und vor dem Reduzierschritt (b) ein Schritt zur selektiven Freisetzung von ATP aus der zweiten Zellpopulation erfolgt, wobei die zweite Zellpopulation Wirtszellen von einem Tier sind, aus dem die Probe stammt.

7. Verfahren nach einem der Ansprüche 1 bis 6, wobei das Verfahren den Schritt des Zugebens eines Apyrasehemmers nach dem Reduzierschritt umfasst.

8. Verfahren nach einem der Ansprüche 1 bis 3 oder 7, wobei die Probe eine Zellkulturmedium-Probe, Nahrungsmittelprobe, eine Getränkeprobe, eine Arzneimittelprobe, eine Abwasserprobe, eine Umweltprobe wie ein Abstrich von einer Oberfläche, oder eine Trinkwasserprobe ist.

9. Verfahren nach Anspruch 1, wobei das Verfahren einen Verfahrensablauf zur Sequenzierung durch Synthese umfasst, die verunreinigenden Nucleotide überschüssige dNTP oder Analoga davon umfassen, die nach einem abgeschlossenen Sequenzierungszyklus vorhanden sind, und die an der Probe durchgeführte Analyse ein Ablesen der Sequenz ist.

10. Verfahren zur Durchführung einer Pyrosequenzierung, das folgende Schritte umfasst:
a. Durchführen einer Pyrosequenzierungsreaktion, die eine Zugabe eines Nucleosidtriphosphats umfasst;
b. Umsetzen des in Schritt (a) freigewordenen Pyrophosphats zu ATP über eine enzymatische Reaktion;
c. Bestimmen der in Schritt (b) gebildeten ATP-Menge;
d. Abbauen von nicht eingebautem Nucleosidtriphosphat aus Schritt (a) und in Schritt (b) gebildetem ATP mit einem Enzym Apyrase;
e. mindestens einmaliges Wiederholen der Schritte a bis d;
wobei das Enzym Apyrase eine Apyrase von *Shigella flexneri* ist.

11. Verfahren nach einem der vorhergehenden Ansprüche, wobei die Apyrase von *Shigella flexneri* eine Aminosäuresequenz mit einer Sequenzübereinstimmung von mindestens 80% mit SEQ ID NO: 10 umfasst.

12. Verfahren nach einem der vorhergehenden Ansprüche, wobei die Apyrase von *Shigella flexneri* in einem Puffer mit einem pH-Wert im Bereich von 6 bis 9 und einer lonenstärke von mindestens 300 mM bereitgestellt wird.

13. Verwendung einer Apyrase von *Shigella flexneri* zum Abbauen von verunreinigenden Nucleosidtriphosphaten oder Nucleosiddisphosphaten in einem Analyseverfahren.

14. Verwendung nach Anspruch 13, wobei die verunreinigenden Nucleoside verunreinigendes ATP und/oder verunreinigende ATP-Analoga umfassen und das Analyseverfahren ein Assay zum Messen von ATP umfasst.

15. Verwendung einer Apyrase von *Shigella flexneri* in einem Sequenzierungsassay durch Synthese, vorzugsweise einem Pyrosequenzierungsassay.

## Revendications

1. Procédé de réduction de la quantité de nucléosides diphosphates et/ou nucléosides triphosphates contaminants, comprenant les étapes consistant à
a. préparer un échantillon contenant des nucléosides diphosphates et/ou nucléosides triphosphates contaminants, tels que l'ATP et/ou des analogues de l'ATP dont les désoxyribonucléosides triphosphates ;
b. réduire la quantité de nucléosides diphosphates et/ou nucléosides triphosphates contaminants dans l'échantillon avec une enzyme apyrase, ladite enzyme apyrase étant une apyrase de *Shigella flexneri* ; et
c. effectuer une analyse de l'échantillon, ladite analyse comprenant un essai qui aurait été affecté par les nucléosides diphosphates et/ou nucléosides triphosphates contaminants s'ils n'avaient pas été réduits dans l'étape b.

2. Procédé de détermination de la quantité d'ATP dans un échantillon, comprenant les étapes consistant à :
a. préparer un échantillon contenant de l'ATP et/ou des analogues de l'ATP contaminants dont les désoxyribonucléosides triphosphates ;
b. réduire la quantité d'ATP et/ou d'analogues de l'ATP contaminants dans l'échantillon par dégradation avec une enzyme apyrase ;
c. rendre l'ATP à déterminer disponible pour la détermination ; et
d. déterminer la quantité d'ATP à déterminer dans l'échantillon, l'enzyme apyrase étant une apyrase de *Shigella flexneri.*

3. Procédé de détermination de la quantité d'ATP présent dans une première population de cellules dans un échantillon, comprenant les étapes consistant à :
a. préparer un échantillon contenant de l'ATP et/ou des analogues de l'ATP contaminants dont les désoxyribonucléosides triphosphates ;
b. réduire la quantité d'ATP et/ou d'analogues de l'ATP contaminants dans l'échantillon par dégradation avec une enzyme apyrase ;
c. libérer l'ATP à déterminer de la première population de cellules ; et
d. déterminer la quantité d'ATP libéré ; l'enzyme apyrase de l'étape (b) étant une apyrase de *Shigella flexneri.*

4. Procédé selon la revendication 3, dans lequel l'étape de libération (b) implique la lyse de la première population de cellules ; la première population de cellules comprend des cellules bactériennes ; et dans lequel l'échantillon est un échantillon biologique d'un animal ou d'un être humain.

5. Procédé selon l'une quelconque des revendications 1 à 4, dans lequel l'échantillon est un échantillon de sang, un échantillon de plasma, un échantillon de sérum, un échantillon d'urine, un échantillon fécal, ou un prélèvement provenant d'un patient.

6. Procédé selon l'une quelconque des revendications 2 à 5, dans lequel au moins une fraction de l'ATP contaminant est présente dans une seconde population de cellules, et l'étape de réduction (b) est précédée par une étape consistant à libérer sélectivement de l'ATP de la seconde population de cellules, la seconde population de cellules étant des cellules hôtes d'un animal duquel l'échantillon est tiré.

7. Procédé selon l'une quelconque des revendications 1 à 6, le procédé comprenant l'étape consistant à ajouter un inhibiteur d'apyrase après l'étape de réduction.

8. Procédé selon l'une quelconque des revendications 1 à 3 ou 7, dans lequel l'échantillon est un échantillon de milieu de culture cellulaire, un échantillon d'aliment, un échantillon de boisson, un échantillon pharmaceutique, un échantillon d'eaux usées, un échantillon environnemental tel qu'un prélèvement d'une surface ou un échantillon d'eau potable.

9. Procédé selon la revendication 1, dans lequel le procédé comprend une procédure de séquençage par synthèse, les nucléotides contaminants comprennent des dNTP en excès ou des analogues de ceux-ci présents après un cycle de séquençage terminé, et l'analyse effectuée sur l'échantillon est une lecture de séquence.

10. Procédé de pyroséquençage comprenant les étapes consistant à :
a. effectuer une réaction de pyroséquençage comprenant l'ajout d'un nucléoside triphosphate ;
b. convertir le pyrophosphate libéré dans l'étape (a) en ATP par le biais d'une réaction enzymatique ;
c. déterminer la quantité d'ATP formé dans l'étape (b) ;
d. mettre en œuvre une dégradation des nucléosides triphosphates non incorporés de l'étape (a) et de l'ATP formé dans l'étape (b) avec une enzyme apyrase ;
e. répéter les étapes a à d au moins une fois ;
dans lequel l'enzyme apyrase est une apyrase de *Shigella flexneri.*

11. Procédé selon l'une quelconque des revendications précédentes, dans lequel l'apyrase de *Shigella flexneri* comprend une séquence d'acides aminés avec au moins 80 % d'identité de séquence à la SEQ. ID No: 10.

12. Procédé selon l'une quelconque des revendications précédentes, dans lequel l'apyrase de *Shigella flexneri* est fournie dans un tampon ayant un pH compris entre 6 et 9 et une force ionique d'au moins 300 mM.

13. Utilisation d'une apyrase de *Shigella flexneri* pour dégrader des nucléosides triphosphates ou nucléosides diphosphates contaminants dans un procédé analytique.

14. Utilisation selon la revendication 13, dans laquelle les nucléosides contaminants comprennent de l'ATP et/ou des analogues de l'ATP contaminants, et le procédé analytique comprend un essai pour mesurer l'ATP.

15. Utilisation d'une apyrase de *Shigella flexneri* dans un essai de séquençage par synthèse, de préférence un essai de pyroséquençage.


### SEQUENCE LISTING

<110> ApiRays AB
<120> ANALYTICAL AND DIAGNOSTIC METHODS UTILIZING SHIGELLA FLEXNERI
   APYRASE
<130> NP0155WO
<150> SE 1451332-9
   <151> 2014-11-07
<160> 10
<170> PatentIn version 3.5
<210> 1
   <211> 1365
   <212> DNA
   <213> Solanum tuberosum
<400> 1
   atgttgaacc aaaatagtca ttttgttttc acaattttgg caatattttt ggttttgccc      60
   ctaagtttat tatccaaaaa tgtggatgct caaattccat taagaagaca tttattaagt     120
   catgaatcag aacattatgc agtaatattt gatgctggaa gtactggaag tagagttcat     180
   gtttttcgat ttgatgaaaa attaggactt cttcctattg gcaacaatat tgagtatttt     240
   atggcgacag agccaggttt aagttcatat gcagaagatc caaaggctgc agccaattca     300
   cttgagccac ttttagatgg agctgaagga gttgttcctc aagaattgca atctgaaaca     360
   cctttagaac ttggggcaac agcaggtctt aggatgttaa aaggggatgc agctgaaaaa     420
   attctacaag cagtgagaaa tttagtgaag aatcaaagca ctttccatag caaagatcaa     480
   tgggtcacta ttcttgatgg aactcaagaa ggctcttata tgtgggctgc aataaattat     540
   ttattgggaa atttgggcaa agattataaa agtacaacag caacaattga tcttggtggt     600
   ggttcagtcc aaatggcata tgctatatca aatgaacaat ttgcaaaagc tcctaataat     660
   gaggatggag aaccttatgt acaacaaaaa catcttatgt caaaagatta taatctctat     720
   gtacatagtt atttaaacta tgggcaatta gcaggtcgag ctgagatttt caaggcttca     780
   agaaatgaaa gtaatccttg tgctctggaa ggatatgatg ggtattactc atatggagga     840
   gtggactaca aagtaaaagc accaaagaaa ggtagtagtt ggaagagatg caggaggtta     900
   actagacatg cacttaaaat aaatgcaaaa tgcaagattg aagaatgcac cttcaatgga     960
   gtgtggaatg gtggtggtgg tgatggacaa aaaaatattc atgcttcatc atttttttat    1020
   gatattggtg ctcaggttgg cattgttgac accaaatttc catctgctct agcaaagcca    1080
   attcaatact taaatgcagc taaagttgct tgccaaacaa atgtggcaga tataaaatcc    1140
   atattcccaa aaactcaaga tagaaatatc ccatacttat gtatggactt gatatatgag    1200
   tacactttgc ttgttgatgg atttggacta aatccacaca aagaaataac agtgatacat    1260
   gatgtgcaat acaaaaacta tctagttgga gcagcatggc cattgggatg tgccattgac    1320
   ttggtttctt caactacaaa caaaattaga gttgcatcat cttaa                    1365
<210> 2
   <211> 1134
   <212> DNA
   <213> Shigella flexneri
<400> 2
   tcacaaatca tcataatcaa gagacaaaac gatacgaaaa aataagataa aaaacatcgt      60
   tcttttacca cattattttc cgtgaatatg aaaaataatg ttattacttt aatataagac     120
   tattttttgt ttttccatca ctctgttcaa atttttccgc atgacttgtg ttttttgtaa    180
   tacagctcgt tttttacagc tgaccaaaat catcaattaa ttatgctaag gaaataaatt     240
   atgaaaacca aaaactttct tcttttttgt attgctacaa atatgatttt tatcccctca    300
   gcaaatgctc tgaaggcaga aggttttctc actcaacaaa cttcaccaga cagtttgtca     360
   atacttccgc cgcctccggc agagaattca gtagtatttc aggctgacaa agctcattat     420
   gaattcggcc gctcgctccg ggatgctaat cgtgtacgtc tcgctagcga agatgcatac     480
   tacgagaatt ttggtcttgc attttcagat gcttatggca tggatatttc aagggaaaat     540
   accccaatct tatatcagtt gttaacacaa gtactacagg atagccatga ttacgccgtg     600
   cgtaacgcca aagaatatta taaaagagtt cgtccattcg ttatttataa agacgcaacc     660
   tgtacacctg ataaagatga gaaaatggct atcactggct cttatccctc tggtcatgca     720
   tcctttggtt gggcagtagc actgatactt gcggagatta atcctcaacg taaagcggaa     780
   atacttcgac gtggatatga gtttggagaa agtcgggtca tctgcggtgc gcattggcaa     840
   agcgatgtag aggctgggcg tttaatggga gcatcggttg ttgcagtact tcataataca     900
   cctgaattta ccaaaagcct tagcgaagcc aaaaaagagt ttgaagaatt aaatactcct     960
   accaatgaac tgaccccata aagctggaca gcctgtatca ggctatggag ggcccataga    1020
   caaatctacc ctatatgagc ataggaggag tctatgggca caccacgttt tacccctgaa   1080
   tttaagggat tactggaaag gctgggacat atcctccggc agaagcagaa aaag          1134
<210> 3
   <211> 780
   <212> DNA
   <213> Shigella flexneri
<400> 3
   gatatacata tgaaaaccaa aaactttctt cttttttgta ttgctacaaa tatgattttt     60
   atcccctcag caaatgctct gaaggcagaa ggttttctca ctcaacaaac ttcaccagac    120
   agtttgtcaa tacttccgcc gcctccggca gagaattcag tagtatttca ggctgacaaa    180
   gctcattatg aattcggccg ctcgctccgg gatgctaatc gtgtacgtct cgctagcgaa      240
   gatgcatact acgagaattt tggtcttgca ttttcagatg cttatggcat ggatatttca      300
   agggaaaata ccccaatctt atatcagttg ttaacacaag tactacagga tagccatgat      360
   tacgccgtgc gtaacgccaa agaatattat aaaagagttc gtccattcgt tatttataaa      420
   gacgcaacct gtacacctga taaagatgag aaaatggcta tcactggctc ttatccctct      480
   ggtcatgcat cctttggttg ggcagtagca ctgatacttg cggagattaa tcctcaacgt      540
   aaagcggaaa tacttcgacg tggatatgag tttggagaaa gtcgggtcat ctgcggtgcg      600
   cattggcaaa gcgatgtaga ggctgggcgt ttaatgggag catcggttgt tgcagtactt      660
   cataatacac ctgaatttac caaaagcctt agcgaagcca aaaaagagtt tgaagaatta      720
   aatactccta ccaatgaact gaccccacat catcaccatc accactaacg tacgatgttc      780
<210> 4
   <211> 259
   <212> PRT
   <213> Shigella flexneri
<400> 4
   Asp Ile His Met Lys Thr Lys Asn Phe Leu Leu Phe Cys Ile Ala Thr
   1               5                   10                  15
   Asn Met Ile Phe Ile Pro Ser Ala Asn Ala Leu Lys Ala Glu Gly Phe
               20                  25                  30
   Leu Thr Gln Gln Thr Ser Pro Asp Ser Leu Ser Ile Leu Pro Pro Pro
           35                  40                  45
   Pro Ala Glu Asn Ser Val Val Phe Gln Ala Asp Lys Ala His Tyr Glu
       50                  55                  60
   Phe Gly Arg Ser Leu Arg Asp Ala Asn Arg Val Arg Leu Ala Ser Glu
   65                  70                  75                  80
   Asp Ala Tyr Tyr Glu Asn Phe Gly Leu Ala Phe Ser Asp Ala Tyr Gly
                   85                  90                  95
   Met Asp Ile Ser Arg Glu Asn Thr Pro Ile Leu Tyr Gln Leu Leu Thr
               100                 105                 110
   Gln Val Leu Gln Asp Ser His Asp Tyr Ala Val Arg Asn Ala Lys Glu
           115                 120                 125
   Tyr Tyr Lys Arg Val Arg Pro Phe Val Ile Tyr Lys Asp Ala Thr Cys
       130                 135                 140
   Thr Pro Asp Lys Asp Glu Lys Met Ala Ile Thr Gly Ser Tyr Pro Ser
   145                 150                 155                 160
   Gly His Ala Ser Phe Gly Trp Ala Val Ala Leu Ile Leu Ala Glu Ile
                   165                 170                 175
   Asn Pro Gln Arg Lys Ala Glu Ile Leu Arg Arg Gly Tyr Glu Phe Gly
               180                 185                 190
   Glu Ser Arg Val Ile Cys Gly Ala His Trp Gln Ser Asp Val Glu Ala
           195                 200                 205
   Gly Arg Leu Met Gly Ala Ser Val Val Ala Val Leu His Asn Thr Pro
       210                 215                 220
   Glu Phe Thr Lys Ser Leu Ser Glu Ala Lys Lys Glu Phe Glu Glu Leu
   225                 230                 235                 240
   Asn Thr Pro Thr Asn Glu Leu Thr Pro His His His His His His Arg
                   245                 250                 255
   Thr Met Phe
<210> 5
   <211> 28
   <212> DNA
   <213> Artificial
<220>
   <223> Primer
<400> 5
   cgcggatccc tgaaggcaga aggttttc 28
<210> 6
   <211> 27
   <212> DNA
   <213> Artificial
<220>
   <223> Primer
<400> 6
   cccaagcttt tatggggtca gttcatt 27
<210> 7
   <211> 53
   <212> DNA
   <213> Artificial
<220>
   <223> Primer
<400> 7
   gaacatcgta cgttagtggt gatggtgatg atgtggggtc agttcattgg tag 53
<210> 8
   <211> 28
   <212> DNA
   <213> Artificial
<220>
   <223> Primer
<400> 8
   gatatacata tgaaaaccaa aaactttc 28
<210> 9
   <211> 454
   <212> PRT
   <213> Solanum tuberosum
<400> 9
   Met Leu Asn Gln Asn Ser His Phe Ile Phe Ile Ile Leu Ala Ile Phe
   1               5                   10                  15
   Leu Val Leu Pro Leu Ser Leu Leu Ser Lys Asn Val Asn Ala Gln Ile
               20                  25                  30
   Pro Leu Arg Arg His Leu Leu Ser His Glu Ser Glu His Tyr Ala Val
           35                  40                  45
   Ile Phe Asp Ala Gly Ser Thr Gly Ser Arg Val His Val Phe Arg Phe
       50                  55                  60
   Asp Glu Lys Leu Gly Leu Leu Pro Ile Gly Asn Asn Ile Glu Tyr Phe
   65                  70                  75                  80
   Met Ala Thr Glu Pro Gly Leu Ser Ser Tyr Ala Glu Asp Pro Lys Ala
                   85                  90                  95
   Ala Ala Asn Ser Leu Glu Pro Leu Leu Asp Gly Ala Glu Gly Val Val
               100                 105                 110
   Pro Gln Glu Leu Gln Ser Glu Thr Pro Leu Glu Leu Gly Ala Thr Ala
           115                 120                 125
   Gly Leu Arg Met Leu Lys Gly Asp Ala Ala Glu Lys Ile Leu Gln Ala
       130                 135                 140
   Val Arg Asn Leu Val Lys Asn Gln Ser Thr Phe His Ser Lys Asp Gln
   145                 150                 155                 160
   Trp Val Thr Ile Leu Asp Gly Thr Gln Glu Gly Ser Tyr Met Trp Ala
                   165                 170                 175
   Ala Ile Asn Tyr Leu Leu Gly Asn Leu Gly Lys Asp Tyr Lys Ser Thr
               180                 185                 190
   Thr Ala Thr Ile Asp Leu Gly Gly Gly Ser Val Gln Met Ala Tyr Ala
           195                 200                 205
   Ile Ser Asn Glu Gln Phe Ala Lys Ala Pro Gln Asn Glu Asp Gly Glu
       210                 215                 220
   Pro Tyr Val Gln Gln Lys His Leu Met Ser Lys Asp Tyr Asn Leu Tyr
   225                 230                 235                 240
   Val His Ser Tyr Leu Asn Tyr Gly Gln Leu Ala Gly Arg Ala Glu Ile
                   245                 250                 255
   Phe Lys Ala Ser Arg Asn Glu Ser Asn Pro Cys Ala Leu Glu Gly Cys
               260                 265                 270
   Asp Gly Tyr Tyr Ser Tyr Gly Gly Val Asp Tyr Lys Val Lys Ala Pro
           275                 280                 285
   Lys Lys Gly Ser Ser Trp Lys Arg Cys Arg Arg Leu Thr Arg His Ala
       290                 295                 300
   Leu Lys Ile Asn Ala Lys Cys Asn Ile Glu Glu Cys Thr Phe Asn Gly
   305                 310                 315                 320
   Val Trp Asn Gly Gly Gly Gly Asp Gly Gln Lys Asn Ile His Ala Ser
                   325                 330                 335
   Ser Phe Phe Tyr Asp Ile Gly Ala Gln Val Gly Ile Val Asp Thr Lys
               340                 345                 350
   Phe Pro Ser Ala Leu Ala Lys Pro Ile Gln Tyr Leu Asn Ala Ala Lys
           355                 360                 365
   Val Ala Cys Gln Thr Asn Val Ala Asp Ile Lys Ser Ile Phe Pro Lys
       370                 375                 380
   Thr Gln Asp Arg Asn Ile Pro Tyr Leu Cys Met Asp Leu Ile Tyr Glu
   385                 390                 395                 400
   Tyr Thr Leu Leu Val Asp Gly Phe Gly Leu Asn Pro His Lys Glu Ile
   405 410 415
   Thr Val Ile His Asp Val Gln Tyr Lys Asn Tyr Leu Val Gly Ala Ala
   420 425 430
   Trp Pro Leu Gly Cys Ala Ile Asp Leu Val Ser Ser Thr Thr Asn Lys
   435 440 445
   Ile Arg Val Ala Ser Ser
   450
<210> 10
   <211> 246
   <212> PRT
   <213> Shigella flexneri
<400> 10
   Met Lys Thr Lys Asn Phe Leu Leu Phe Cys Ile Ala Thr Asn Met Ile
   1               5                   10                  15
   Phe Ile Pro Ser Ala Asn Ala Leu Lys Ala Glu Gly Phe Leu Thr Gln
               20                  25                  30
   Gln Thr Ser Pro Asp Ser Leu Ser Ile Leu Pro Pro Pro Pro Ala Glu
           35                  40                  45
   Asn Ser Val Val Phe Gln Ala Asp Lys Ala His Tyr Glu Phe Gly Arg
       50                  55                  60
   Ser Leu Arg Asp Ala Asn Arg Val Arg Leu Ala Ser Glu Asp Ala Tyr
   65                  70                  75                  80
   Tyr Glu Asn Phe Gly Leu Ala Phe Ser Asp Ala Tyr Gly Met Asp Ile
                   85                  90                  95
   Ser Arg Glu Asn Thr Pro Ile Leu Tyr Gln Leu Leu Thr Gln Val Leu
               100                 105                 110
   Gln Asp Ser His Asp Tyr Ala Val Arg Asn Ala Lys Glu Tyr Tyr Lys
           115                 120                 125
   Arg Val Arg Pro Phe Val Ile Tyr Lys Asp Ala Thr Cys Thr Pro Asp
       130                 135                 140
   Lys Asp Glu Lys Met Ala Ile Thr Gly Ser Tyr Pro Ser Gly His Ala
   145                 150                 155                 160
   Ser Phe Gly Trp Ala Val Ala Leu Ile Leu Ala Glu Ile Asn Pro Gln
                       165                 170                 175
   Arg Lys Ala Glu Ile Leu Arg Arg Gly Tyr Glu Phe Gly Glu Ser Arg
               180                 185                 190
   Val Ile Cys Gly Ala His Trp Gln Ser Asp Val Glu Ala Gly Arg Leu
           195                 200                 205
   Met Gly Ala Ser Val Val Ala Val Leu His Asn Thr Pro Glu Phe Thr
       210                 215                 220
   Lys Ser Leu Ser Glu Ala Lys Lys Glu Phe Glu Glu Leu Asn Thr Pro
   225                 230                 235                 240
   Thr Asn Glu Leu Thr Pro
                   245